# EUROPEAN PATENT APPLICATION

(11) **EP 4 410 966 A1**
(43) Date of publication of application: **07.08.2024**
(21) Application number: 22875116.0
(22) Date of filing: 29.09.2022
(51) Int. Cl.: C12N 5/10, C07K 14/705

(54) **CHIMERIC ANTIGEN RECEPTOR IMMUNE CELL, AND PREPARATION METHOD THEREFOR AND APPLICATION THEREOF**

(30) Priority: 29.09.2021 CN 202111155155
(71) Applicant: Gracell Biotechnologies (Shanghai) Co., Ltd., Shanghai 200233 (CN)
(72) Inventor: DONG, Qi, Shanghai 200233 (CN); YIN, Wenjie, Shanghai 200233 (CN); SHEN, Lianjun, Shanghai 200233 (CN); CAO, Wei, Shanghai 200233 (CN)
(74) Representative: Dehns
(86) International application number: PCT/CN2022/122839
(87) International publication number: WO 2023/051735

(57) **Abstract**

Provided are a chimeric antigen receptor immune cell, and a preparation method therefor and an application thereof. The surface of the chimeric antigen receptor immune cell expresses a receptor targeting a specific antigen, and also expresses a signal conversion protein. The signal conversion protein is a fusion protein containing a dominant negative receptor TGFBR2 extracellular element and an IL-7Rα intracellular element, and the transmembrane region of the signal conversion protein is from any source. The chimeric antigen receptor immune cell can further convert, by means of the signal conversion protein, the inhibitory signal of a TGF-β immunosuppressive factor in a tumor microenvironment that is not conducive to the survival of immune cells into a cytokine activation signal, thereby significantly enhancing the survival of the immune cells and having a more sustained tumor killing effect.

## Description

### Technical field

The present invention relates to the field of immune cell therapy, and more specifically to an engineered immune cell targeting solid tumors and hematological malignancies with the function of converting tumor microenvironment inhibitory signals into supportive signals.

### Background

Currently, lymphocyte therapy mainly includes αβT cell therapy and NK cell therapy, including autologous and allogeneic immunotherapy.

Autologous immunotherapy uses the patient's immune cells, while allogeneic immunotherapy uses the immune cells of an allogeneic subject (such as a healthy person). After *in vitro* culture, gene editing, and amplification of the required immune cells, they are reintroduced into the patient's body, allowing these cells to recognize tumor cells in a non-MHC restricted manner, activate and enhance the body's immunity, kill tumor cells, and achieve the goal of treating or alleviating the disease.

Several immunotherapies including LAK, DC, CIK, DC-CIK, TCR-T, CAR-T, NK, CAR-NK, etc., have been developed for tumor associated antigen (TAA) targets. Among them, CAR-T cell immunotherapy includes autologous and universal CAR-T therapies. Universal CAR-T has advantages such as allogeneity and being off-the-shelf. The market prospects for using universal CAR-T to treat hematological malignancies and solid tumors are very promising, but there are still many problems that need to be solved. At present, immunotherapy has shown outstanding efficacy in hematological malignancies, but its efficacy is not ideal in solid tumors. The main reasons include three aspects: firstly, lacking safe tumor specific antigen targets; secondly, heterogeneity of solid tumors; thirdly, the existence of complex tumor cell microenvironment factors, including the presence of immunosuppressive cells such as Tregs and MDSCs, as well as immunosuppressive factors, which are not conducive to the survival of immune cells and thus inhibiting the effectiveness of immunotherapy.

In summary, there is an urgent need to develop more effective and more sustained immunotherapies targeting solid tumors in this field.

### Summary of the invention

The purpose of the present invention is to provide a more effective and more sustained chimeric antigen receptor immune cell that can target solid tumors and hematological malignancies, as well as preparation method therefor and application thereof.

In the first aspect of the present invention, it provides an engineered immune cell, wherein the engineered immune cell is a T cell or NK cell, and the immune cell has the following characteristics:
(a) the immune cell expresses a chimeric antigen receptor (CAR), wherein the CAR targets surface markers of tumor cells; and
(b) the immune cell expresses an exogenous signal conversion protein, which is located on the cell membrane of the immune cell and has an extracellular domain of an immunosuppressive receptor, a transmembrane region (or a transmembrane domain), and an intracellular domain of an immunostimulatory receptor.

In another preferred embodiment, the immunosuppressive receptor is a TGF-β receptor.

In another preferred embodiment, the immunostimulatory receptor is selected from the group consisting of: IL-7 receptor, IL-15 receptor, IL-12 receptor, IL-2 receptor, IL-18 receptor, IL-21 receptor, and a combination thereof.

In another preferred embodiment, the immunostimulatory receptor is an IL-7 receptor.

In another preferred embodiment, the transmembrane region is derived from an immunosuppressive receptor, an immunostimulatory receptor, and a combination thereof.

In another preferred embodiment, the transmembrane region is not derived from immunosuppressive receptors or immunostimulatory receptors.

In another preferred embodiment, the transmembrane region and extracellular domain are derived from the same immunosuppressive receptor or from different immunosuppressive receptors.

In another preferred embodiment, the transmembrane region and intracellular domain are derived from the same immunostimulatory receptor or from different immunostimulatory receptors.

In another preferred embodiment, the immune cell comprises autologous or allogeneic αβT cells, γδT cells, NKT cells, NK cells, and a combination thereof.

In another preferred embodiment, the engineered immune cell is selected from the group consisting of:
(i) chimeric antigen receptor αβT cells (CAR-T cells);
(ii) chimeric antigen receptor γδT cells (CAR-T cells);
(iii) chimeric antigen receptor NKT cells (CAR-NKT cells);
(iv) chimeric antigen receptor NK cells (CAR-NK cells).

In another preferred embodiment, the amino acid sequence of the CAR is as shown in any one of SEQ ID NOs: 8-17.

In another preferred embodiment, the amino acid sequence of the CAR is as shown in any one of SEQ ID NOs: 8-11 and SEQ ID NOs: 16-17.

In another preferred embodiment, the engineered immune cell of the first aspect of the present invention, characterized in that the CAR has a structure as shown in formula Ia or Ib:

L1-scFv-H-TM1-C-CD3ζ (Ia)

L1-scFv-H-TM1-C-CD3ζ-(A-S)n1-A-E-(A-S)n2 (Ib)

wherein,
L1 is absent or a signal peptide sequence;
scFv is a single chain variable domain of targeting antibodies;
H is absent or a hinge region;
TM1 is a transmembrane domain;
C is a costimulatory signaling domain;
CD3ζ is a cytoplasmic signaling sequence derived from CD3ζ (including wild-type or mutants/modifiers thereof);
A is a self-cleaving 2A peptide;
S is another exogenous recombinant protein;
n1 or n2 each independently is 0 or 1;
E is a signal conversion protein;
"-" is a linker or peptide bond.

In another preferred embodiment, L1 is a signal peptide of a protein selected from the group consisting of: CD8, GM-CSF, CD4, CD28, CD137, or mutants/modifiers thereof, and a combination thereof.

In another preferred embodiment, L1 is the signal peptide of CD8 protein.

In another preferred embodiment, the scFv targets mesothelin, Claudin18.2, MUC1, GPC3, PSCA, Her2, and a combination thereof, respectively.

In another preferred embodiment, the scFv targets P4 human specific mesothelin.

In another preferred embodiment, H is a hinge region of a protein selected from the group consisting of: CD8, CD28, CD137, IgG, and a combination thereof.

In another preferred embodiment, H is the hinge region of CD8 protein.

In another preferred embodiment, TM1 is a transmembrane region of a protein selected from the group consisting of: CD28, CD3 epsilon, CD45, CD4, CD5, CD8, CD9, CD16, CD22, CD33, CD37, CD64, CD80, CD86, CD134, CD137, CD154, CD278, CD152, CD279, CD233, or mutants/modifiers thereof, and a combination thereof.

In another preferred embodiment, TM1 is the transmembrane region of CD28 protein.

In another preferred embodiment, C is a costimulatory signaling domain of a protein selected from the group consisting of: OX40, CD2, CD7, CD27, CD28, CD30, CD40, CD70, CD134, 4-1BB (CD137), PD-1, Dap10, LIGHT, NKG2C, B7-H3, ICAM-1, LFA-1 (CD11a/CD18), ICOS (CD278), NKG2D, GITR, OX40L, 2B4, TLR, or mutants/modifiers thereof, and a combination thereof.

In another preferred embodiment, C is the costimulatory signaling domain of CD28 protein.

In another preferred embodiment, A is P2A, T2A, or a combination thereof;

In another preferred embodiment, S is selected from the group consisting of: IL-15 mutant - IL-15Ra, mbIL-15, IL-12p40, IL-2, IL-18, IL-21, etc., and a combination thereof.

In another preferred embodiment, S is an IL-15 mutant - IL-15Ra.

In another preferred embodiment, S has an amino acid sequence as shown in SEQ ID NO: 1.

In another preferred embodiment, E may be constitutive expression or inducible expression.

In another preferred embodiment, the engineered immune cell of the first aspect of the present invention, characterized in that the signal conversion protein has a structure as shown in formula V:

L2-Z1-TM2-Z2 (V)

wherein,
L2 is absent or a signal peptide sequence;
Z1 is an extracellular domain of an immunosuppressive receptor;
TM2 is a transmembrane region;
Z2 is an intracellular domain of an immunostimulatory receptor;
"-" is a linker or peptide bond.

In another preferred embodiment, the immunosuppressive receptor is selected from the group consisting of: TGF-β receptor and subtypes thereof, and a combination thereof (including wild-type or mutants/modifiers thereof).

In another preferred embodiment, the TGF-β receptor is a TGFBR2 receptor.

In another preferred embodiment, the TGFBR2 receptor comprises: TGFBR2 receptor isoform A or TGFBR2 receptor isoform B.

In another preferred embodiment, L2 is a signal peptide sequence of an immunosuppressive receptor.

In another preferred embodiment, L2 is the signal peptide sequence of the TGFBR2 receptor.

In another preferred embodiment, L2 has an amino acid sequence as shown in SEQ ID NO: 2.

In another preferred embodiment, Z1 is an extracellular domain of a TGF-β receptor selected from the group consisting of: TGFBR2 receptor isoform A and TGFBR2 receptor isoform B.

In another preferred embodiment, Z1 has an amino acid sequence as shown in SEQ ID NO: 3 or SEQ ID NO: 4.

In another preferred embodiment, TM2 is a transmembrane region of a receptor selected from the group consisting of: IL-7 receptor, IL-15 receptor, IL-12 receptor, IL-2 receptor, IL-18 receptor, IL-21 receptor, and a combination thereof.

In another preferred embodiment, TM2 is the transmembrane region of IL-7 receptor and subtypes thereof, and a combination thereof (including wild-type or mutants/modifiers thereof).

In another preferred embodiment, the IL-7 receptor is IL-7Rα_{∘}

In another preferred embodiment, TM2 is selected from the transmembrane region of IL-7Rα, or an IL-7Rα mutant.

In another preferred embodiment, TM2 is selected from the group consisting of: PRTR transmembrane region, IL-7Rα transmembrane region, TPOR transmembrane region.

In another preferred embodiment, TM2 has an amino acid sequence as shown in SEQ ID NO: 5, SEQ ID NO: 6, SEQ ID NO: 22, SEQ ID NO: 23, or SEQ ID NO: 24.

In another preferred embodiment, the immunostimulatory receptor is selected from the group consisting of: IL-7 receptor and subtypes thereof, and a combination thereof (including wild-type or mutants/modifiers thereof).

In another preferred embodiment, the IL-7 receptor is IL-7Rα.

In another preferred embodiment, Z2 is the intracellular domain of IL-7Rα.

In another preferred embodiment, Z2 has an amino acid sequence as shown in SEQ ID NO: 7.

In another preferred embodiment, the engineered immune cell of the first aspect of the present invention, characterized in that the amino acid sequence encoding the signal conversion protein is as shown in position 523-933 of SEQ ID NO: 8, position 523-908 of SEQ ID NO: 9, position 523-936 of SEQ ID NO: 10, position 523-911 of SEQ ID NO: 11, position 937-1347 of SEQ ID NO: 16, position 937-1350 of SEQ ID NO: 17, SEQ ID NO: 19, SEQ ID NO: 20 or SEQ ID NO: 21.

In another preferred embodiment, it provides a chimeric antigen receptor T cell (CAR-T cell), wherein the CAR-T cell has one or more of the following features:
(a) the cell expresses a chimeric antigen receptor CAR, wherein the CAR targets surface markers of tumor cells; and
(b) when the CAR-T cell contacts with an inducer, the CAR-T cell induces the expression of a signal conversion protein.

In another preferred embodiment, in the CAR cell, the CAR and the signal conversion protein are expressed in tandem.

In another preferred embodiment, in the CAR cell, the CAR and the signal conversion protein are independently expressed.

In another preferred embodiment, the "activation" refers to the binding of the CAR to surface markers of tumor cells.

In another preferred embodiment, the "surface markers of tumor" refers to specific antigens on the tumor surface.

In another preferred embodiment, the chimeric antigen receptor CAR is located on the cell membrane of the engineered immune cell.

In another preferred embodiment, the chimeric antigen receptor CAR is located on the cell membrane of the CAR-T cell.

In another preferred embodiment, the signal conversion protein is located on the cell membrane of the CAR-T cell.

In the second aspect of the present invention, it provides a method for preparing the engineered immune cell of the first aspect of the present invention, which comprises the following steps of:
(a) providing an immune cell to be engineered; and
(b) engineering the immune cell to express the CAR molecule and exogenous signal conversion protein, thereby obtaining the engineered immune cell of the first aspect of the present invention.

In another preferred embodiment, step (b) comprises:
(b1) introducing a first expression vector expressing the CAR into the immune cell; and (b2) introducing a second expression vector expressing the signal conversion protein into the immune cell;
wherein the step (b1) can be performed before or after step (b2), or simultaneously or alternately with step (b2).

In another preferred embodiment, the first expression vector and the second expression vector are the same expression vector or different expression vectors.

In another preferred embodiment, the expression vector comprises a viral vector or a plasmid.

In another preferred embodiment, the CAR has the structure as shown in formula Ia or Ib.

In another preferred embodiment, when the immune cell to be engineered in step (a) has already expressed CAR, then in step (b), the method comprises a step of (b2) introducing a second expression vector expressing the signal conversion protein into the immune cell.

In another preferred embodiment, the transcription directions of the first expression vector and the second expression vector are in the same direction (→ →), face-to-face direction (→ ←), or back-to-back direction (←→).

In another preferred embodiment, the first expression vector and the second expression vector are located on the same vector or different vectors.

In another preferred embodiment, the first expression vector and the second expression vector are located on the same vector.

In another preferred embodiment, when the first and second expression vectors are located on the same vector, there is also a third expression vector for expressing a self-cleaving 2A peptide between the first and second expression vectors.

In another preferred embodiment, the self-cleaving 2A peptide is P2A, T2A, or a combination thereof.

In another preferred embodiment, the vector is a viral vector, preferably the viral vector contains the first and second expression vectors in a tandem form.

In another preferred embodiment, the vector is selected from the group consisting of: DNA, RNA, plasmid, lentiviral vector, adenoviral vector, retroviral vector, transposon, other gene transfer systems, and a combination thereof.

In another preferred embodiment, the vector is the FUW series lentivirus vector.

In the third aspect of the present invention, it provides a formulation comprising the engineered immune cell of the first aspect of the present invention, and a pharmaceutically acceptable carrier, diluent, or excipient.

In another preferred embodiment, the formulation comprises the CAR-T cell of the present invention, and a pharmaceutically acceptable carrier, diluent, or excipient.

In another preferred embodiment, the formulation is a liquid formulation.

In another preferred embodiment, the dosage form of the formulation includes an injection.

In another preferred embodiment, the concentration of the engineered immune cell (such as CAR-T cell) in the formulation is 1 × 10³ - 1 × 10⁸ cells / mL, preferably 1 × 10⁴ - 1 × 10⁷ cells / mL.

In the fourth aspect of the present invention, it provides use of the engineered immune cell of the first aspect of the present invention, in the manufacture of a medicament or formulation for preventing and/or treating a cancer or tumor.

In another preferred embodiment, it provides use of the CAR-T cell of the first aspect of the present invention, in the manufacture of a medicament or formulation for preventing and/or treating a cancer or tumor.

In another preferred embodiment, the formulation contains CAR-T cells, and a pharmaceutically acceptable carrier, diluent, or excipient.

In another preferred embodiment, the tumor is selected from the group consisting of: solid tumor, hematological malignancy, lymphoma, and a combination thereof.

In another preferred embodiment, the tumor expresses markers selected from the group consisting of: mesothelin, Claudin18.2, MUC1, GPC3, PSMA, Her2, 5T4, EGFR, CD20, NY-ESO-1, and a combination thereof.

In another preferred embodiment, the tumor is selected from the group consisting of: ovarian cancer, lung cancer, pancreatic cancer, liver cancer, gastric cancer, breast cancer, mesothelioma, etc.

In the fifth aspect of the present invention, it provides a kit for preparing the engineered immune cell of the first aspect of the present invention, wherein the kit comprises a container and the following located inside the container:
(1) a first nucleic acid sequence containing a first expression cassette for expressing the CAR; and
(2) a second nucleic acid sequence containing a second expression cassette for co-expressing a signal conversion protein.

In another preferred embodiment, it provides a kit for preparing the CAR-T cell of the present invention, wherein the kit comprises a container and the following located inside the container:
(1) a first nucleic acid sequence containing a first expression cassette for expressing the CAR; and
(2) a second nucleic acid sequence containing a second expression cassette for co-expressing a signal conversion protein.

In another preferred embodiment, the first and second nucleic acid sequences are independent or connected.

In another preferred embodiment, the first and second nucleic acid sequences are located in the same container or different containers.

In another preferred embodiment, the first and second nucleic acid sequences are located on the same vector or different vectors.

In another preferred embodiment, the first and second nucleic acid sequences are located on the same vector.

In another preferred embodiment, when the first and second nucleic acid sequences are located in the same vector, there is also a third nucleic acid sequence between the first and second nucleic acid sequences, which contains a third expression cassette for expressing a self-cleaving 2A peptide.

In another preferred embodiment, the self-cleaving 2A peptide is P2A, T2A, or a combination thereof.

In another preferred embodiment, the vector is a viral vector, preferably the viral vector contains first and second nucleic acid sequences in tandem form.

In the sixth aspect of the present invention, it provides a recombinant signal conversion protein, which is a membrane protein located on the cell membrane and has an extracellular domain of an immunosuppressive receptor, a transmembrane region (or a transmembrane domain), and an intracellular domain of an immunostimulatory receptor.

In another preferred embodiment, the signal conversion protein is defined as in the first aspect of the present invention.

In the seventh aspect of the present invention, it provides a nucleotide sequence encoding the signal conversion protein of the sixth aspect of the present invention.

In the eighth aspect of the present invention, it provides a vector comprising the nucleotide sequence of the seventh aspect of the present invention.

In the ninth aspect of the present invention, it provides a host cell comprising the vector of the eighth aspect of the present invention, or having the nucleotide sequence of the seventh aspect of the present invention integrated into its genome.

In the tenth aspect of the present invention, it provides a method for preparing the signal conversion protein of the sixth aspect of the present invention, characterized in that it comprises the step of:
(a) culturing the host cell of the ninth aspect of the present invention, thereby expressing the signal conversion protein of the sixth aspect of the present invention.

It should be understood that within the scope of the present invention, the above-mentioned technical features of the present invention and the technical features specifically described in the following (such as the examples) can be combined with each other to form a new or preferred technical solution, which are not redundantly repeated one by one due to space limitation.

### Description of the drawings

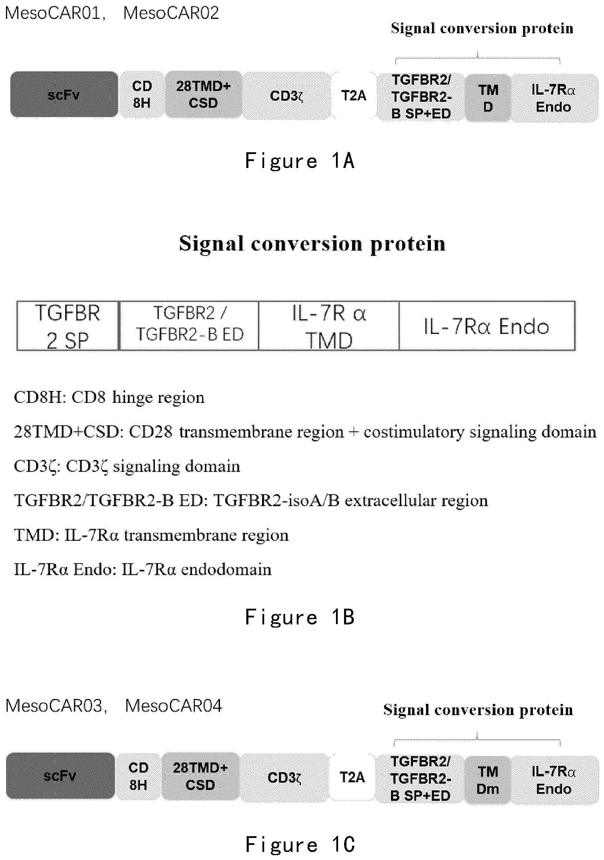

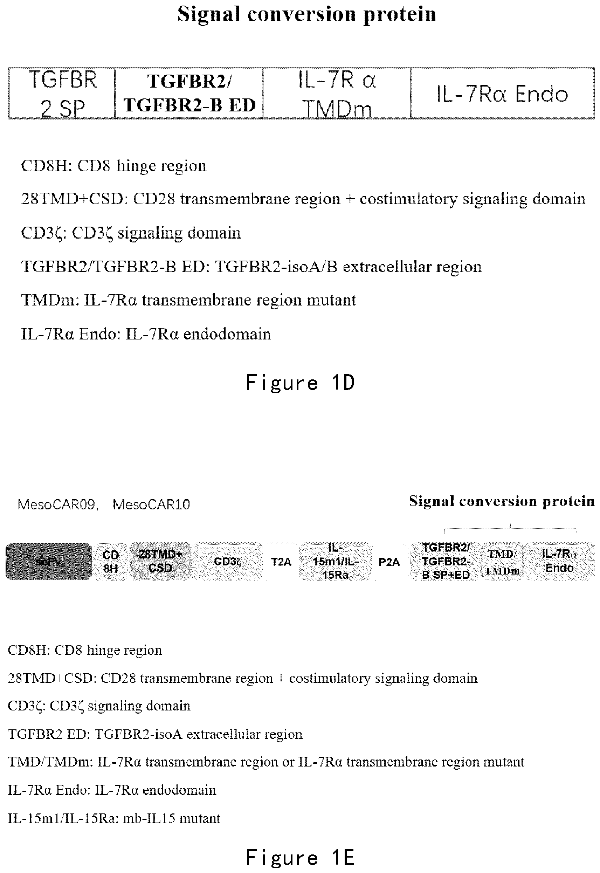

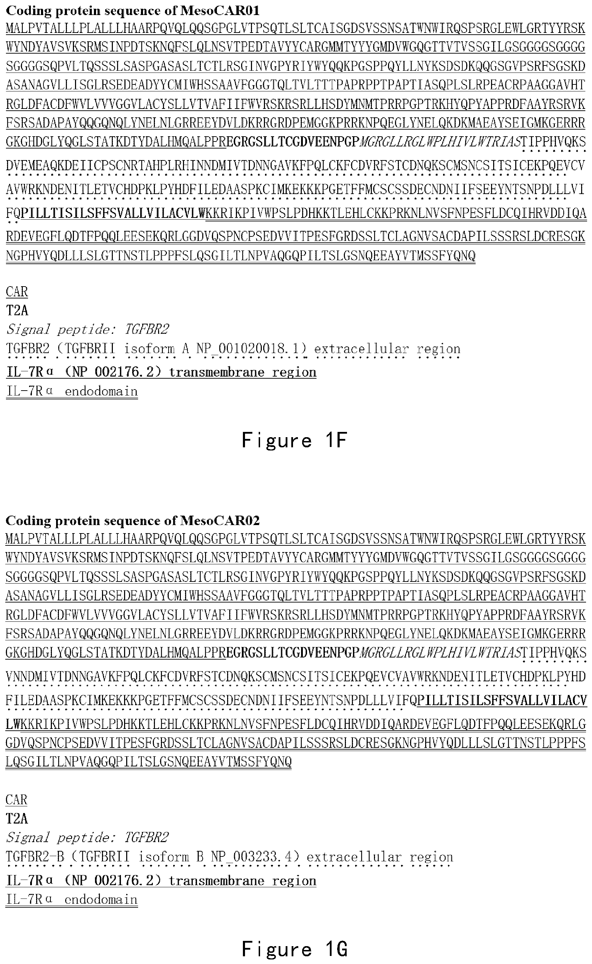

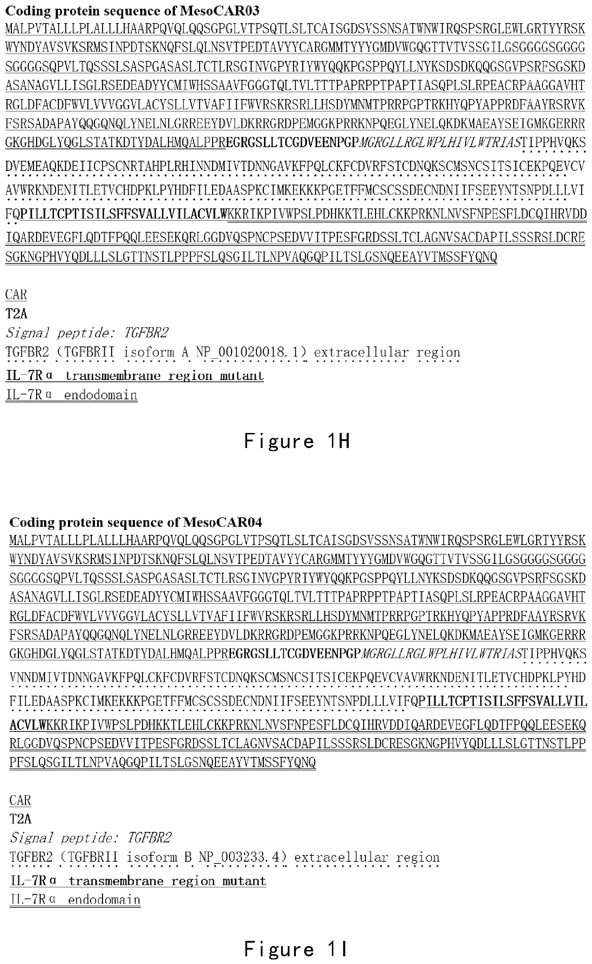

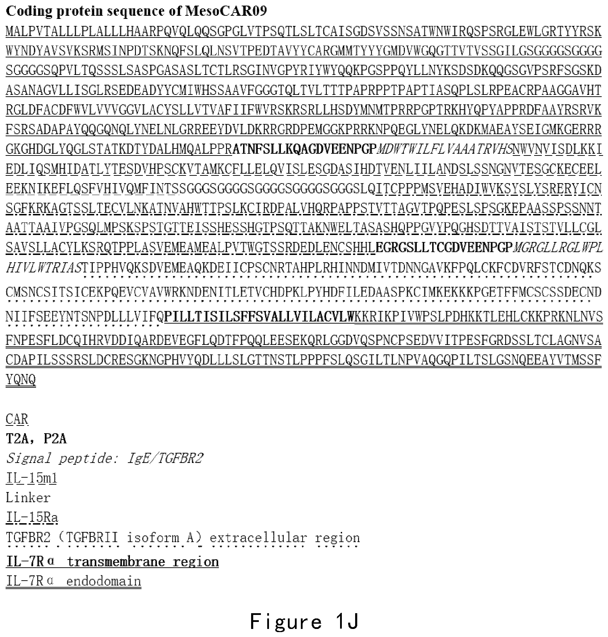

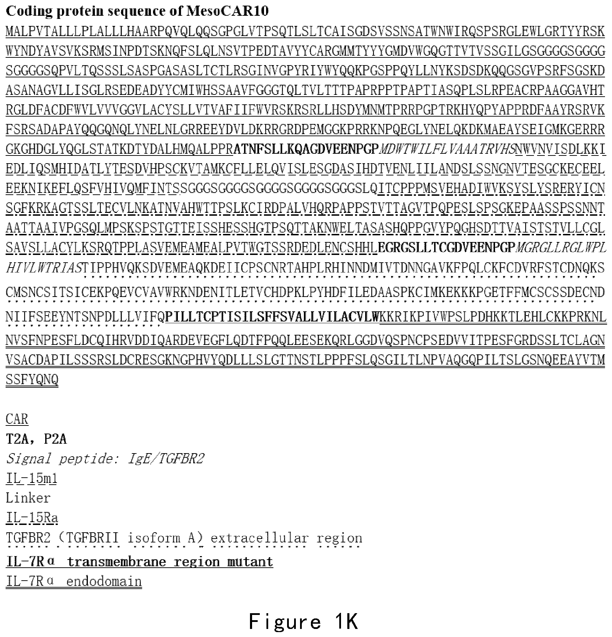

Figure 1 illustrates a schematic diagram of the protein sequences encoded by the MesoCAR01, 02, 03, 04, 09, 10, and E1m1 plasmids. Among them, taking Figure 1F as an example, the CAR sequence is underlined, T2A is bold, the signal peptide TGFBR2 is italicized, the extracellular region of TGFBR2 (TGFBRII isoform A NP_001020018.1) is marked with an emphasis sign (.), the transmembrane region of IL-7Rα (NP_002176.2) is marked in bold and by underlining, and the intracellular region of IL-7Rα is marked with double underline. Figures 1G-1L are represented in a similar way.
Figure 2 illustrates the expression of CAR-T surface proteins (MSLN, TGFBR2, IL-15R).
Figure 3 illustrates the expansion, survival rate of the CAR-T cells, and expression of CARs after freeze-thaw.
Figure 4 illustrates the effect of *in vitro* instant killing (RTCA method, Target: OVCAR3 and HCC70).
Figure 5 illustrates the result of pSTAT5 signaling (+/- TGF-β1).
Figure 6 illustrates the analysis of CAR-T cell apoptosis after co-culturing with target cells, and the results of Annexin V staining are shown in the figure.
Figure 7 illustrates the effect of *in vitro* pharmacological experiments - multi-round killing (A. design scheme, B. killing efficacy, C. expression of CAR⁺ T cells, D. proliferation efficacy of CAR⁺ T cells).
Figure 8 illustrates the effect of *in vitro* pharmacological experiments - multi-round killing (+TGF-β1 Target: MDA-MB231-E10) (A. design scheme, B. the fourth and fifth rounds of continuous *in vitro* killing experiments: cell killing efficacy, expression of CARs and proliferation efficacy of CAR⁺ T cells, C. comparison of proliferation efficacy of different CAR-T cells under multi-round killing, D. PD-1 expression).
Figure 9 illustrates the effect of the *in vivo* pharmacological experiment (OVCAR3). TGI stands for tumor growth inhibition.
Figure 10 illustrates the inhibitory effects of MesoCAR01, MesoCAR02, and MesoCAR05 on the TGFβ / Smad signaling pathway under different concentrations of TGF-β1, TGF-β2, or TGF-β3 treatment.
Figure 11 illustrates the effects of MesoCAR01, MesoCAR03, CAR11, CAR12, and CAR13 on the TGFβ / Smad and STATS signaling pathways under different concentrations of TGF-β1 treatment.

### Detailed description

After extensive and intensive research and massive screening, the inventors have co-expressed the CAR structure and signal conversion proteins (such as the CAR structure together with TGFBR2 extracellular region and IL-7Rα intracellular region) in CAR-T cells for the first time. *In vitro* and *in vivo* experiments have shown that the extracellular structure of the dominant inactivated receptor TGFBR2 can effectively block or reduce TGF-β signal transduction, protect CAR-T cells, and combat the effects of immunosuppressive environments; and the IL-7Rα intracellular domain can further convert inhibitory signals into cytokine signals, and improve CAR-T cell proliferation, cellular exhaustion and persistence, thereby increasing therapeutic effects.

Compared with the prior art, the immune cells of the present invention can recognize target antigens (such as various target antigens on the surface of tumor cells such as ovarian cancer, lung cancer, pancreatic cancer, breast cancer, etc., including but not limited to: mesothelin, Claudin18.2, MUC1, GPC3, PSCA, Her2, etc.) through CAR, so as to target the lesions of solid tumors, hematological malignancies, lymphomas, etc.; at the same time, the inhibitory signals of TGF-β immunosuppressive factors, which are not conducive to the survival of immune cells in the tumor microenvironment, can be further converted into cytokine activation signals via signal conversion proteins, thereby enhancing the survival of immune cells and the sustained effects of tumor killing, and reducing tumor recurrence. On this basis, the present invention has been completed.

Taking CAR-T cells as an example, a representative and detailed explanation of the engineered immune cells of the present invention is provided. The engineered immune cells of the present invention are not limited to the CAR-T cells described in the context. The engineered immune cells of the present invention have the same or similar technical features and beneficial effects as the CAR-T cells described in the context. Specifically, when immune cells express a chimeric antigen receptor CAR, NK cells are equivalent to T cells (or the T cells can be replaced with NK cells).

### Terms

In order to make it easier to understand the present disclosure, certain terms are first defined. As used herein, each of the following terms shall have the meanings given below unless expressly provided herein.

The term "about" may refer to a value or composition within an acceptable error range of a particular value or composition determined by those of ordinary skill in the art, which will depend in part on how the value or composition is measured or determined.

The term "administration" refers to the physical introduction of the product of the present invention into a subject using any one of various methods and delivery systems known to those skilled in the art, including intravenous, intratumor, intramuscular, subcutaneous, intraperitoneal, spinal or other parenteral routes of administration, such as by injection or infusion.

The terms "transmembrane domain" and "transmembrane region" can be used interchangeably to refer to a connecting sequence between the extracellular and intracellular domains of a membrane protein, typically the transmembrane region is located entirely or mostly within the cell membrane. The transmembrane regions of common membrane proteins are well known or can be determined using conventional methods.

The terms "TGF-β receptor", "TGF-βR", "TGFBR" and "TGFBR receptor" can be used interchangeably to refer to transforming growth factor - β receptors. Many cell surfaces have TGF-β receptors exist in three forms: type I, II, and III, among which type I and II TGF-βR are both glycoproteins, and type III receptor is a proteoglycan.

### Antibody

As used herein, the term "antibody" (Ab) shall include, but is not limited to an immunoglobulin, that specifically binds to an antigen and comprises at least two heavy (H) chains and two light (L) chains interconnected by disulfide bonds, or an antigen-binding portion thereof. Each H chain contains a heavy chain variable region (abbreviated herein as VH) and a heavy chain constant region. The heavy chain constant region contains three constant domains, CH1, CH2 and CH3. Each light chain contains a light chain variable region (abbreviated herein as VL) and a light chain constant region. The light chain constant region contains a constant domain CL. The VH and VL regions can be further subdivided into hypervariable regions called complementary determination regions (CDR), which are interspersed within more conservative regions called frame regions (FR). Each VH and VL contains three CDRs and four FRs, which are arranged from amino terminus to carboxyl terminus in the following order: FR1, CDR1, FR2, CDR2, FR3, CDR3, FR4. The variable regions of heavy and light chains contain binding domains that interact with an antigen.

### Antigen binding domain

As used herein, the "antigen binding domain" and "single-chain antibody fragment" refer to a Fab fragment, a Fab' fragment, a F(ab')₂ fragment, or a single Fv fragment that has antigen-binding activity. The Fv antibody contains a heavy chain variable region and a light chain variable region of an antibody, but has no constant region, and the Fv antibody is the smallest antibody fragment with all antigen-binding sites. Generally, the Fv antibody also includes a polypeptide linker between the VH and VL domains, and can form the structure required for antigen binding. The antigen binding domain is usually a scFv (single-chain variable fragment). The single-chain antibody is preferably an amino acid chain sequence encoded by a nucleotide chain.

### Tumor associated antigen

Taking mesothelin (MSLN) as an example, mesothelin is a common tumor associated antigen (TAA). It has been reported that mesothelin is highly expressed in ovarian cancer, lung cancer, pancreatic cancer and breast cancer cells, and is limited in normal tissues. As an effective immunotherapy target, there have been reports on immunotherapy methods such as antibodies against mesothelin and chimeric antigen antibodies targeting mesothelin.

### Chimeric antigen receptor (CAR)

As used herein, the chimeric immune antigen receptor (CAR) comprises an extracellular domain, an optional hinge region, a transmembrane domain, and an intracellular domain. The extracellular domain comprises an optional signal peptide and a target-specific binding domain (also known as an antigen binding domain). The intracellular domain includes a costimulatory domain and a CD3ζ chain moiety. When the CAR is expressed in T cells, the extracellular region can recognize a specific antigen, and then transduce this signal through the intracellular domain, causing the cell activation and proliferation, cytolytic toxicity, and secretion of cytokines, such as IL-2 and IFN-γ and so on. This affects tumor cells, causing them to not grow, be prompted to die, or be affected in other ways, and leading to a reduction or elimination of the patient's tumor burden. The antigen binding domain is preferably fused to one or more of the intracellular domains from the costimulatory molecule and the CD3ζ chain.

### Chimeric antigen receptor T cell (CAR-T cell)

As used herein, the terms "CAR-T cell", "CAR-T", "CAR-T cell of the present invention" all refer to the CAR-T cell of the first aspect of the present invention. The CAR-T cell of the present invention can be used to treat tumors with high expression of mesothelin, Claudin18.2, MUC1, GPC3, PSCA, Her2, etc., such as ovarian cancer, lung cancer, pancreatic cancer, breast cancer, and the like.

CAR-T cells have the following advantages over other T-cell-based treatments: (1) the action process of CAR-T cells is not restricted by MHC; (2) since many tumor cells express same tumor antigen, once the construction of a CAR gene targeting a certain tumor antigen is completed, it can be widely used; (3) CAR can use both tumor protein antigens and glycolipid non-protein antigens, thereby expanding the target range of tumor antigens; (4) the use of patient's autologous cells reduces the risk of rejection reaction; (5) CAR-T cells have the immune memory function and can survive *in vivo* for a long time.

### Chimeric antigen receptor NK cell (CAR-NK cell)

As used herein, the terms "CAR-NK cell", "CAR-NK", "CAR-NK cell of the present invention" all refer to the CAR-NK cell of the first aspect of the present invention. The CAR-NK cell of the present invention can be used to treat tumors with high expression of mesothelin, Claudin18.2, MUC1, GPC3, PSCA, Her2, etc., such as ovarian cancer, lung cancer, pancreatic cancer, breast cancer, and the like.

Natural killer (NK) cells are a major class of immune effector cells that protect the body from viral infection and invasion of tumor cells through non-antigen-specific pathways. Engineered (genetically modified) NK cells may obtain new functions, including the ability to specifically recognize tumor antigens and enhanced anti-tumor cytotoxicity.

Compared with autologous CAR-T cells, CAR-NK cells also have the following advantages, for example: (1) they directly kill tumor cells by releasing perforin and granzyme, but have no killing effect on normal cells of the body; (2) they release a small amount of cytokines, thus reducing the risk of cytokine storms; (3) they are easy to expand *in vitro,* and can be developed into "off-the-shelf" products. In addition to these, they are similar to CAR-T cell therapy.

### TGF-β and receptors thereof

As used herein, the term "TGF-β" refers to the transforming growth factor beta. It should be understood that the term includes wild-type and mutant TGF-β. In the present invention, the TGF-β includes TGF-β of both human and non-human mammals.

As used herein, the term "TGF-β receptor" refers to the transforming growth factor beta receptor.

It has been confirmed that tumor cells such as ovarian cancer, breast cancer and prostate cancer secrete large amounts of TGF-β cytokines. As immunosuppressive factors, TGF-β cytokines can significantly inhibit the activation and proliferation of T cells, thus promoting their differentiation into Tregs, and reducing their effector functions.

### Signal conversion protein

As used herein, the terms "signaling convertor (abbreviated as Sc)", "signal conversion receptor", or "signal conversion protein of the present invention" can be used interchangeably and all refer to a fusion protein composed of the extracellular domain of the dominant inactivated receptor TGFBR2 and the intracellular element of IL-7Rα protein, which are expressed in tandem. Surprisingly, the signal conversion protein of the present invention can convert the inhibitory signals of TGF-β immunosuppressive factor in a tumor microenvironment that is not conducive to the survival of immune cells, into an activation signal, thereby enhancing the survival of the immune cells and tumor killing sustained effect, improving the effectiveness of immunotherapy, and reducing tumor recurrence.

According to the different types of TGFBR2 extracellular domains and transmembrane regions connecting the extracellular and intracellular domains, i.e. the different combinations of Z1 and TM2 in formula V, signal conversion proteins are classified into different types or subtypes. As shown in Table 1, based on the combination of Z1 and TM2, some representative types include (but are not limited to):

**Table 1**

| Signal conversion protein type | Z1 type | TM2 type |
|---|---|---|
| Sc1-type signal conversion protein | TGFBR2 isoA | IL-7Rα |
| Sc2-type signal conversion protein | TGFBR2 isoB | IL-7Rα |
| Sc3-type signal conversion protein | TGFBR2 isoA | IL-7Rα mutant |
| Sc4-type signal conversion protein | TGFBR2 isoB | IL-7Rα mutant |

### Expression cassette

As used herein, "expression cassette" or "expression cassette of the present invention" includes the first expression cassette and the second expression cassette. The expression cassette of the present invention is described in the fifth aspect of the present invention. The first expression cassette comprises a nucleic acid sequence encoding the CAR. The second expression cassette expresses an exogenous signal conversion protein.

In the present invention, the signal conversion protein can be expressed in constitutive or inducible forms.

In the case of induced expression, when the CAR-T cells are activated by the corresponding inducer, the second expression cassette expresses the signal conversion protein; in this way, when CAR-T cells of the present invention are not in contact with the corresponding inducer, the second expression cassette does not express the signal conversion protein.

In one embodiment, the first expression cassette and the second expression cassette each further comprises a promoter and/or a terminator. The promoter of the second expression cassette can be either a constitutive or inducible prompter.

### Vector

The present invention also provides a vector containing the expression cassette of the present invention. Vectors derived from retroviruses such as the lentivirus are suitable tools to achieve long-term gene transfer since they allow long-term, stable integration of a transgene and its propagation in daughter cells. Lentiviral vectors have the advantage over vectors derived from onco-retroviruses such as murine leukemia viruses in that they can transduce non-proliferating cells. They also have the advantage of low immunogenicity.

Usually, the expression cassette or nucleic acid sequence of the present invention is typically linked to downstream of a promoter, and incorporated into an expression vector. The vectors can be integrated into the genome of eukaryotic cells and replicated accordingly. Typical cloning vectors contain transcription and translation terminators, initiation sequences, and promoters useful for regulation of the expression of the desired nucleic acid sequence.

The expression vectors of the present invention may also be used for nucleic acid immune and gene therapy, using standard gene delivery protocols. Methods for gene delivery are known in the art. See, e.g., U.S, Pat. Nos. 5,399,346, 5,580,859, 5,589,466, incorporated by reference herein in their entireties.

The expression cassette or the nucleotide sequence can be cloned into a number of types of vectors. For example, the expression cassette or the nucleotide sequence can be cloned into a vector including, but not limited to a plasmid, a phagemid, a phage derivative, an animal virus, and a cosmid. Vectors of particular interest include expression vectors, replication vectors, etc.

Further, the expression vector may be provided to a cell in the form of a viral vector. Viral vector technology is well known in the art and is described, for example, in Molecular Cloning: A Laboratory Manual (Sambrook et al, Cold Spring Harbor Laboratory, New York, 2001), and in other virology and molecular biology manuals. Viruses, which are useful as vectors include, but are not limited to, retroviruses, adenoviruses, adeno-associated viruses, herpes viruses, and lentiviruses. In general, a suitable vector contains an origin of replication functional in at least one organism, a promoter sequence, convenient restriction endonuclease sites, and one or more selectable markers, (e.g., WO 01/96584; WO 01/29058; and U.S, Pat. No. 6,326,193).

A number of viral based systems have been developed for gene transfer into mammalian cells. For example, retroviruses provide a convenient platform for gene delivery systems. A selected gene can be inserted into a vector and packaged into retroviral particles using techniques known in the art. The recombinant virus can then be isolated and delivered to cells of the subject either *in vivo* or *ex vivo.* A number of retroviral systems are known in the art. In one embodiment, lentiviral vectors are used. A number of DNA virus systems are known in the art. In some embodiments, adenovirus vectors are used. A number of adenovirus vectors are known in the art.

Additional promoter elements, e.g., enhancers, can regulate the frequency of transcriptional initiation. Typically, they are located in the region 30-110 bp upstream of the initiation site, although a number of promoters have recently been shown to contain functional elements downstream of the initiation site as well. The spacing between promoter elements is frequently flexible, so that promoter function is preserved when elements are inverted or moved relative to one another. In the thymidine kinase (tk) promoter, the spacing between promoter elements can be increased to 50 bp apart before activity begins to decline. Depending on the promoter, it appears that individual elements can function either cooperatively or independently to activate transcription.

One example of a suitable promoter is the cytomegalovirus (CMV) promoter sequence. This promoter sequence is a strong constitutive promoter sequence capable of driving high levels of expression of any polynucleotide sequence operatively linked thereto. Another example of a suitable promoter is Elongation Growth Factor-1α (EF-1α). However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, avian leukemia virus promoter, Epstein-Barr virus (EBV) immediate early promoter, Rous sarcoma virus promoter, as well as human gene promoters such as, but not limited to, the actin promoter, the myosin promoter, the hemoglobin promoter, and the creatine kinase promoter. Further, the present invention should not be limited to the use of constitutive promoters, inducible promoters are also contemplated as part of the present invention. The use of an inducible promoter provides a molecular switch capable of turning on expression of the polynucleotide sequence which it is operatively linked when such expression is desired, or turning off the expression when expression is not desired. Examples of inducible promoters include, but are not limited to a metallothionein promoter, a glucocorticoid promoter, a progesterone promoter, and a tetracycline promoter.

The expression vector to be introduced into a cell can also contain either a selectable marker gene or a reporter gene or both to facilitate identification and selection of expressing cells from the population of cells sought to be transfected or infected through viral vectors. In other aspects, the selectable marker may be carried on a separate piece of DNA and used in a co-transfection procedure. Both selectable markers and reporter genes may be flanked with appropriate regulatory sequences to enable expression in the host cells. Useful selectable markers include, for example, antibiotic-resistance genes, such as neomycin and the like.

Methods of introducing and expressing genes into a cell are known in the art. In the context of an expression vector, the vector can be readily introduced into a host cell, e.g., mammalian (such as human T cell), bacterial, yeast, or insect cell by any method in the art. For example, the expression vector can be transferred into a host cell by physical, chemical, or biological means.

Physical methods for introducing a polynucleotide into a host cell include calcium phosphate precipitation, cationic complex transfection, lipofection, particle bombardment, microinjection, electroporation, and the like. Methods for producing cells comprising vectors and/or exogenous nucleic acids are well-known in the art. See, for example, Molecular Cloning: A Laboratory Manual (Sambrook et al, Cold Spring Harbor Laboratory, New York, 2001). A preferred method for the introduction of a polynucleotide into a host cell is lipofection and cationic complex polyethyleneimine transfection.

Biological methods for introducing a polynucleotide into a host cell include the use of DNA and RNA vectors. Viral vectors, and especially retroviral vectors, have become the most widely used method for inserting genes into mammalian, e.g., human cells. Other viral vectors can be derived from lentivirus, poxviruses, herpes simplex virus I, adenoviruses and adeno-associated viruses, and the like. For example, see U.S. Pat, Nos. 5,350,674 and 5,585,362.

Chemical means for introducing a polynucleotide into a host cell include colloidal dispersion systems, such as macromolecule complexes, nanocapsules, microspheres, and beads; and lipid-based systems including oil-in-water emulsions, micelles, mixed micelles, and liposomes. An exemplary colloidal system for use as a delivery vehicle *in vitro* and *in vivo* is a liposome (e.g., an artificial membrane vesicle).

In the case where a non-viral delivery system is utilized, an exemplary delivery vehicle is a liposome. The use of lipid formulations is contemplated for the introduction of the nucleic acids into a host cell (*in vitro, ex vivo* or *in vivo*)*.* In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

In a preferred embodiment of the present invention, the vector is a lentiviral vector.

### Formulation

The present invention provides a formulation comprising the engineered immune cell (such as the CAR-T cell) of the first aspect of the present invention, and a pharmaceutically acceptable carrier, diluent or excipient. In one embodiment, the formulation is a liquid formulation. Preferably, the formulation is an injection. Preferably, the concentration of the CAR-T cell in the formulation is 1 × 10³ - 1 × 10⁸ cells / mL, more preferably 1 × 10⁴ - 1 × 10⁷ cells / mL.

In one embodiment, the formulation may comprises buffers such as neutral buffered saline, phosphate buffered saline and the like; carbohydrates such as glucose, mannose, sucrose or glucan, mannitol; proteins; polypeptides or amino acids such as glycine; antioxidants; chelating agents such as EDTA or glutathione; adjuvants (e.g., aluminum hydroxide); and preservatives. The formulation of the present invention is preferably formulated for intravenous administration.

### Therapeutic application

The present invention comprises therapeutic applications using cells (e.g., T cells) transduced with a vector (such as a lentiviral vector) comprising the expression cassette of the present invention. The transduced T cells can target the tumor cell surface markers and express signal conversion proteins, thereby synergistically and significantly increasing the killing efficiency against tumor cells.

Thus, the present invention also provides a method for stimulating a T cell-mediated immune response to a target cell population or tissue in a mammal, which comprises the step of: administering CAR-T cells of the present invention to a mammal.

In one embodiment, the present invention comprises a class of cell therapies, wherein autologous T cells from a patient (or heterologous donor) are isolated, activated and genetically modified to generate CAR-T cells, and then injected into the same patient. In this way, the probability of graft versus host disease is extremely low, and antigens are recognized by T cells in a non-MHC-restricted manner. In addition, one kind of CAR-T can treat all cancers that express the antigen. Unlike antibody therapies, CAR-T cells are able to replicate *in vivo* resulting in long-term persistence that can lead to sustained tumor control

In one embodiment, the CAR-T cells of the present invention can undergo stable *in vivo* expansion and can persist for several months to years. In addition, the CAR-mediated immune response may be part of an adoptive immunotherapy approach in which the CAR-T cells can induce a specific immune response against tumor cells with high expression of antigens recognized by antigen binding domains in the CAR. For example, the CAR-T cells of the present invention induce a specific immune response against tumor cells with high expression of NKG2D ligands.

Cancers that can be treated include tumors that are not vascularized or basically have not yet been vascularized, and tumors that are vascularized. Cancer types that can be treated by the CAR of the present invention include but are not limited to: ovarian cancer, lung cancer, pancreatic cancer, liver cancer, gastric cancer, breast cancer, etc.

Generally, the activated and expanded cells as described herein can be used for treating and preventing diseases such as tumors. Therefore, the present invention provides a method for treating cancers, which comprises a step of administering a therapeutically effective amount of the CAR-T cells of the present invention, to a subject in need thereof.

The CAR-T cells of the present invention may be administered either alone, or as a pharmaceutical composition in combination with diluents and/or with other components such as IL-2, IL-17 or other cytokines or cell populations. Briefly, the pharmaceutical composition of the present invention may comprise a targeting cell population as described herein, in combination with one or more pharmaceutically or physiologically acceptable carriers, diluents or excipients.

The pharmaceutical composition of the present invention may be administered in a manner appropriate to the disease to be treated (or prevented). The quantity and frequency of administration will be determined by such factors as the condition of the patient, and the type and severity of the patient's disease, or can be determined by clinical trials.

When "an immunologically effective amount", "an anti-tumor effective amount", "a tumor-inhibiting effective amount", or "a therapeutic amount" is indicated, the precise amount of the composition of the present invention to be administered can be determined by a physician with consideration of individual differences in age, weight, tumor size, extent of infection or metastasis, and condition of the patient (subject). A pharmaceutical composition comprising the T cells described herein may be administered at a dosage of 10⁴ to 10⁹ cells / kg body weight, preferably 10⁵ to 10⁷ cells / kg body weight, including all integer values within those ranges. A T cell composition may also be administered multiple times at these dosages. The cells can be administered by using infusion techniques that are commonly known in immunotherapy (see, e.g., Rosenberg et al., New Eng. J. of Med. 319: 1676, 1988). The optimal dosage and treatment regime for a particular patient can readily be determined by one skilled in the art of medicine by monitoring the patient for disease signs and adjusting the treatment accordingly.

The administration of the subject composition may be carried out in any convenient manner, including by aerosol inhalation, injection, ingestion, transfusion, implantation or transplantation. The composition described herein may be administered to a patient subcutaneously, intradermaliy, intratumorally, intranodally, intramedullary, intramuscularly, by intravenous injection, or intraperitoneally. In one embodiment, the T cell composition of the present invention is administered to a patient by intradermal or subcutaneous injection. In another embodiment, the T cell composition of the present invention is preferably administered by intravenous injection. The composition of T cells may be injected directly into a tumor, lymph node, or site of infection.

In certain embodiments of the present invention, cells activated and expanded using the methods described herein, or other methods known in the art where T cells are expanded to therapeutic levels, are administered to a patient in conjunction with (e.g., before, simultaneously or following) any number of relevant treatment modalities, including but not limited to treatment with the following agents: the agents such as antiviral therapy, cidofovir and interleukin-2, cytarabine (also known as ARA-C) or natalizumab treatment for MS patients or efalizumab treatment for psoriasis patients or other treatments for PML patients. In further embodiments, the T cells of the present invention may be used in combination with the following: chemotherapy, radiation, immunosuppressive agents, such as cyclosporin, azathioprine, methotrexate, mycophenolate, and FK506, antibodies, or other immunotherapeutic agents. In a further embodiment, the cell composition of the present invention is administered to a patient in conjunction with (e.g., before, simultaneously or following) bone marrow transplantation, or the use of chemotherapy agents such as, fludarabine, external-beam radiotherapy (XRT), cyclophosphamide. For example, in one embodiment, subjects may undergo standard treatment with high dose chemotherapy followed by peripheral blood stem cell transplantation. In certain embodiments, following the transplant, subjects receive an infusion of the expanded immune cells of the present invention. In an additional embodiment, expanded cells are administered before or after surgery.

The dosage of the above treatments to be administered to a patient will vary with the precise nature of the condition being treated and the recipient of the treatment. The scaling of dosages for human administration can be performed according to art-accepted practices. In general, for each treatment or each course of treatment, 1 × 10⁵ - 1 × 10¹⁰ of the modified T cells of the present invention can be applied to a patient by means of, for example, intravenous reinfusion.

### The main advantages of the present invention

1. The immune cells of the present invention contain both CAR and signal conversion proteins, thereby enabling more efficient activation of immune cells.
2. The signal conversion protein of the present invention can not only effectively block or reduce the inhibitory signals of TGF-β immunosuppressive factors on T cells (including TGF-β inhibition of T cell proliferation and induction of cell apoptosis), and on this basis, it can also further converts the TGF-β epidemic immunosuppressive signals into cytokine activation signals, thereby prolonging the survival time of CAR-T cells *in vivo,* effectively and continuously killing tumor cells, and reducing tumor recurrence.
3. Compared with CAR-T cells without signal conversion proteins, immune cells of the present invention exhibit lower expression of exhaustion marker PD-1 under antigen stimulation, thus reducing immune suppression during tumor treatment, significantly prolonging immune cell survival time, and achieving better therapeutic effects.
4. The intracellular molecules of the conversion proteins co-expressed in CAR-T cells of the present invention can also be other exogenous recombinant protein intracellular domains (such as IL-15R, IL-12Rα, IL-2Rα, IL-18R, IL-21R, etc.), which can convert TGF-β immunosuppressive signals into activation signals, and exert corresponding functions.
5. Taking the signal conversion protein containing extracellular TGFBR2 and IL-7Rα intracellular region for an example, it can not only convert inhibition into activation on its own, but can also be introduced into immune cells together with other exogenous recombinant proteins (such as IL-15 mutant - IL- 15Ra fusion protein, mbIL-15, IL-12p40, IL-2, IL-18, IL-21, etc.), to perform corresponding functions without interfering with the function of the CAR structure. For example, it has no adverse effect on the expansion, survival rate of the CAR-T cells and expression of the CARs, and even has a promoting effect.

The present invention is further explained below in conjunction with specific examples. It should be understood that these examples are only for illustrating the present invention and not intend to limit the scope of the present invention. The experimental method without detailed conditions specified in the following embodiments is generally in accordance with conventional conditions, such as those described in Sambrook et al., Molecular Cloning: A Laboratory Manual (New York: Cold Spring Harbor Laboratory Press, 1989), or in accordance with the conditions recommended by the manufacturers. Unless otherwise stated, percentages and parts are calculated by weight.

### Materials and methods

### CAR molecules and structures thereof

In the examples, the constructed CAR molecules are all Meso-CAR, i.e. CAR molecules targeting mesothelin. The compositions of Meso-CAR molecules are selected from the following structures: signal peptide [abbreviated as SP], single-chain variable domain targeting mesothelin [abbreviated as P4scFv], CD8 hinge region [abbreviated as CD8H] and CD28 transmembrane domain [abbreviated as 28TMD], CD28 costimulatory signaling domain [abbreviated as 28CSD], CD3ζ cytoplasmic signaling sequence [abbreviated as CD3ζ], self-cleaving 2A peptide (P2A, T2A, or a combination thereof), signal conversion protein [abbreviated as Sc-type] (Sc1-type, Sc2-type, Sc3-type, Sc4-type), signal peptide sequence of an immunosuppressive receptor (TGFBR2 SP), extracellular domain of an immunosuppressive receptor (TGFBR2 isoA, TGFBR2 isoB), related structures of other exogenous recombinant protein, IL-15 mutant - IL-15Ra fusion protein [abbreviated as IL-15m1 / IL-15Ra] (IL-15m1 / IL-15Ra SP: IgE, IL-15m1, linker, IL-15Ra), and a combination thereof.

Among them, the second generation Meso-CAR molecule that does not express any cytokines as a control is named MesoCAR.

The fourth generation Meso-CAR molecule, which expresses the IL-15 mutant - IL-15Ra fusion protein in addition to the second generation Meso-CAR molecule, is named E1m1. On the basis of the second generation Meso-CAR molecule, the fourth generation Meso-CAR molecules that co-express signal conversion proteins or only partially express the structure of the signal conversion protein are named MesoCAR01 - MesoCAR10, according to different types of TGFBR2 extracellular domains and different types of transmembrane regions connecting the extracellular domains and the intracellular domains in their structures from amino terminal to carboxyl terminal, i.e., the different combinations of Z1 and TM2 in formula V. The specific structures of different types of CAR molecules are shown in Table 2 of Example 3.

### Amino Acid Sequences

SEQ ID NO: 1 (S element: IL-15m1 - IL-15Ra) 
MDWTWILFLVAAATRVHSNWVNVISDLKKIEDLIQSMHIDATLYTESDV
HPSCKVTAMKCFLLELQVISLESGDASIHDTVENLIILANDSLSSNGNVTESGC
KECEELEEKNIKEFLQSFVHIVQMFINTSSGGGSGGGGSGGGGSGGGGSGGGS
LQITCPPPMSVEHADIWVKSYSLYSRERYICNSGFKRKAGTSSLTECVLNKAT
NVAHWTTPSLKCIRDPALVHQRPAPPSTVTTAGVTPQPESLSPSGKEPAASSPS
SNNTAATTAAIVPGSQLMPSKSPSTGTTEISSHESSHGTPSQTTAKNWELTASA
SHQPPGVYPQGHSDTTVAISTSTVLLCGLSAVSLLACYLKSRQTPPLASVEME
AMEALPVTWGTSSRDEDLENCSHHL
SEQ ID NO: 2 (L2 element: signal peptide sequence of TGFBR2 receptor)
   MGRGLLRGLWPLHIVLWTRIAS
SEQ ID NO: 3 (Z1 element: selected from the extracellular domain of TGFBR2 receptor isoform A) 
TIPPHVQKSDVEMEAQKDEIICPSCNRTAHPLRHINNDMIVTDNNGAVKF
PQLCKFCDVRFSTCDNQKSCMSNCSITSICEKPQEVCVAVWRKNDENITLETV
CHDPKLPYHDFILEDAASPKCIMKEKKKPGETFFMCSCSSDECNDNIIFSEEYN
TSNPDLLLVIFQ
SEQ ID NO: 4 (Z1 element: selected from the extracellular domain of TGFBR2 receptor isoform B) 
TIPPHVQKSVNNDMIVTDNNGAVKFPQLCKFCDVRFSTCDNQKSCMSNC
SITSICEKPQEVCVAVWRKNDENITLETVCHDPKLPYHDFILEDAASPKCIMKE
KKKPGETFFMCSCSSDECNDNIIFSEEYNTSNPDLLLVIFQ
SEQ ID NO: 5 (TM2 element: selected from the transmembrane region of IL-7Rα)
   PILLTISILSFFSVALLVILACVLW
SEQ ID NO: 6 (TM2 element: selected from the transmembrane region of IL-7Rα mutant)
   PILLTCPTISILSFFSVALLVILACVLW
SEQ ID NO: 7 (Z2 element: intracellular domain of IL-7Rα) 
KKRIKPIVWPSLPDHKKTLEHLCKKPRKNLNVSFNPESFLDCQIHRVDDI
QARDEVEGFLQDTFPQQLEESEKQRLGGDVQSPNCPSEDVVITPESFGRDSSL
TCLAGNVSACDAPILSSSRSLDCRESGKNGPHVYQDLLLSLGTTNSTLPPPFSL
QSGILTLNPVAQGQPILTSLGSNQEEAYVTMSSFYQNQ
SEQ ID NO: 8 (amino acid sequence of MesoCAR01) 
MALPVTALLLPLALLLHAARPQVQLQQSGPGLVTPSQTLSLTCAISGDSV
SSNSATWNWIRQSPSRGLEWLGRTYYRSKWYNDYAVSVKSRMSINPDTSKN
 
QFSLQLNSVTPEDTAVYYCARGMMTYYYGMDVWGQGTTVTVSSGILGSGG
GGSGGGGSGGGGSQPVLTQSSSLSASPGASASLTCTLRSGINVGPYRIYWYQQ
KPGSPPQYLLNYKSDSDKQQGSGVPSRFSGSKDASANAGVLLISGLRSEDEAD
YYCMIWHSSAAVFGGGTQLTVLTTTPAPRPPTPAPTIASQPLSLRPEACRPAA
GGAVHTRGLDFACDFWVLVVVGGVLACYSLLVTVAFIIFWVRSKRSRLLHSD
YMNMTPRRPGPTRKHYQPYAPPRDFAAYRSRVKFSRSADAPAYQQGQNQLY
NELNLGRREEYDVLDKRRGRDPEMGGKPRRKNPQEGLYNELQKDKMAEAY
SEIGMKGERRRGKGHDGLYQGLSTATKDTYDALHMQALPPREGRGSLLTCG
DVEENPGPMGRGLLRGLWPLHIVLWTRIASTIPPHVQKSDVEMEAQKDEIICP
SCNRTAHPLRHINNDMIVTDNNGAVKFPQLCKFCDVRFSTCDNQKSCMSNCS
ITSICEKPQEVCVAVWRKNDENITLETVCHDPKLPYHDFILEDAASPKCIMKE
KKKPGETFFMCSCSSDECNDNIIFSEEYNTSNPDLLLVIFQPILLTISILSFFSVAL
LVILACVLWKKRIKPIVWPSLPDHKKTLEHLCKKPRKNLNVSFNPESFLDCQI
HRVDDIQARDEVEGFLQDTFPQQLEESEKQRLGGDVQSPNCPSEDVVITPESF
GRDSSLTCLAGNVSACDAPILSSSRSLDCRESGKNGPHVYQDLLLSLGTTNST
LPPPFSLQSGILTLNPVAQGQPILTSLGSNQEEAYVTMSSFYQNQ
SEQ ID NO: 9 (amino acid sequence of MesoCAR02) 
MALPVTALLLPLALLLHAARPQVQLQQSGPGLVTPSQTLSLTCAISGDSV
SSNSATWNWIRQSPSRGLEWLGRTYYRSKWYNDYAVSVKSRMSINPDTSKN
QFSLQLNSVTPEDTAVYYCARGMMTYYYGMDVWGQGTTVTVSSGILGSGG
GGSGGGGSGGGGSQPVLTQSSSLSASPGASASLTCTLRSGINVGPYRIYWYQQ
KPGSPPQYLLNYKSDSDKQQGSGVPSRFSGSKDASANAGVLLISGLRSEDEAD
YYCMIWHSSAAVFGGGTQLTVLTTTPAPRPPTPAPTIASQPLSLRPEACRPAA
GGAVHTRGLDFACDFWVLVVVGGVLACYSLLVTVAFIIFWVRSKRSRLLHSD
YMNMTPRRPGPTRKHYQPYAPPRDFAAYRSRVKFSRSADAPAYQQGQNQLY
NELNLGRREEYDVLDKRRGRDPEMGGKPRRKNPQEGLYNELQKDKMAEAY
SEIGMKGERRRGKGHDGLYQGLSTATKDTYDALHMQALPPREGRGSLLTCG
DVEENPGPMGRGLLRGLWPLHIVLWTRIASTIPPHVQKSVNNDMIVTDNNGA
VKFPQLCKFCDVRFSTCDNQKSCMSNCSITSICEKPQEVCVAVWRKNDENITL
ETVCHDPKLPYHDFILEDAASPKCIMKEKKKPGETFFMCSCSSDECNDNIIFSE
EYNTSNPDLLLVIFQPILLTISILSFFSVALLVILACVLWKKRIKPIVWPSLPDHK
KTLEHLCKKPRKNLNVSFNPESFLDCQIHRVDDIQARDEVEGFLQDTFPQQLE
 
ESEKQRLGGDVQSPNCPSEDVVITPESFGRDSSLTCLAGNVSACDAPILSSSRS
LDCRESGKNGPHVYQDLLLSLGTTNSTLPPPFSLQSGILTLNPVAQGQPILTSL
GSNQEEAYVTMSSFYQNQ
SEQ ID NO: 10 (amino acid sequence of MesoCAR03) 
MALPVTALLLPLALLLHAARPQVQLQQSGPGLVTPSQTLSLTCAISGDSV
SSNSATWNWIRQSPSRGLEWLGRTYYRSKWYNDYAVSVKSRMSINPDTSKN
QFSLQLNSVTPEDTAVYYCARGMMTYYYGMDVWGQGTTVTVSSGILGSGG
GGSGGGGSGGGGSQPVLTQSSSLSASPGASASLTCTLRSGINVGPYRIYWYQQ
KPGSPPQYLLNYKSDSDKQQGSGVPSRFSGSKDASANAGVLLISGLRSEDEAD
YYCMIWHSSAAVFGGGTQLTVLTTTPAPRPPTPAPTIASQPLSLRPEACRPAA
GGAVHTRGLDFACDFWVLVVVGGVLACYSLLVTVAFIIFWVRSKRSRLLHSD
YMNMTPRRPGPTRKHYQPYAPPRDFAAYRSRVKFSRSADAPAYQQGQNQLY
NELNLGRREEYDVLDKRRGRDPEMGGKPRRKNPQEGLYNELQKDKMAEAY
SEIGMKGERRRGKGHDGLYQGLSTATKDTYDALHMQALPPREGRGSLLTCG
DVEENPGPMGRGLLRGLWPLHIVLWTRIASTIPPHVQKSDVEMEAQKDEIICP
SCNRTAHPLRHINNDMIVTDNNGAVKFPQLCKFCDVRFSTCDNQKSCMSNCS
ITSICEKPQEVCVAVWRKNDENITLETVCHDPKLPYHDFILEDAASPKCIMKE
KKKPGETFFMCSCSSDECNDNIIFSEEYNTSNPDLLLVIFQPILLTCPTISILSFFS
VALLVILACVLWKKRIKPIVWPSLPDHKKTLEHLCKKPRKNLNVSFNPESFLD
CQIHRVDDIQARDEVEGFLQDTFPQQLEESEKQRLGGDVQSPNCPSEDVVITP
ESFGRDSSLTCLAGNVSACDAPILSSSRSLDCRESGKNGPHVYQDLLLSLGTT
NSTLPPPFSLQSGILTLNPVAQGQPILTSLGSNQEEAYVTMSSFYQNQ
SEQ ID NO: 11(amino acid sequence of MesoCAR04) 
MALPVTALLLPLALLLHAARPQVQLQQSGPGLVTPSQTLSLTCAISGDSV
SSNSATWNWIRQSPSRGLEWLGRTYYRSKWYNDYAVSVKSRMSINPDTSKN
QFSLQLNSVTPEDTAVYYCARGMMTYYYGMDVWGQGTTVTVSSGILGSGG
GGSGGGGSGGGGSQPVLTQSSSLSASPGASASLTCTLRSGINVGPYRIYWYQQ
KPGSPPQYLLNYKSDSDKQQGSGVPSRFSGSKDASANAGVLLISGLRSEDEAD
YYCMIWHSSAAVFGGGTQLTVLTTTPAPRPPTPAPTIASQPLSLRPEACRPAA
GGAVHTRGLDFACDFWVLVVVGGVLACYSLLVTVAFIIFWVRSKRSRLLHSD
YMNMTPRRPGPTRKHYQPYAPPRDFAAYRSRVKFSRSADAPAYQQGQNQLY
 
NELNLGRREEYDVLDKRRGRDPEMGGKPRRKNPQEGLYNELQKDKMAEAY
SEIGMKGERRRGKGHDGLYQGLSTATKDTYDALHMQALPPREGRGSLLTCG
DVEENPGPMGRGLLRGLWPLHIVLWTRIASTIPPHVQKSVNNDMIVTDNNGA
VKFPQLCKFCDVRFSTCDNQKSCMSNCSITSICEKPQEVCVAVWRKNDENITL
ETVCHDPKLPYHDFILEDAASPKCIMKEKKKPGETFFMCSCSSDECNDNIIFSE
EYNTSNPDLLLVIFQPILLTCPTISILSFFSVALLVILACVLWKKRIKPIVWPSLP
DHKKTLEHLCKKPRKNLNVSFNPESFLDCQIHRVDDIQARDEVEGFLQDTFPQ
QLEESEKQRLGGDVQSPNCPSEDVVITPESFGRDSSLTCLAGNVSACDAPILSS
SRSLDCRESGKNGPHVYQDLLLSLGTTNSTLPPPFSLQSGILTLNPVAQGQPIL
TSLGSNQEEAYVTMSSFYQNQ
SEQ ID NO: 12 (amino acid sequence of MesoCAR05) 
MALPVTALLLPLALLLHAARPQVQLQQSGPGLVTPSQTLSLTCAISGDSV
SSNSATWNWIRQSPSRGLEWLGRTYYRSKWYNDYAVSVKSRMSINPDTSKN
QFSLQLNSVTPEDTAVYYCARGMMTYYYGMDVWGQGTTVTVSSGILGSGG
GGSGGGGSGGGGSQPVLTQSSSLSASPGASASLTCTLRSGINVGPYRIYWYQQ
KPGSPPQYLLNYKSDSDKQQGSGVPSRFSGSKDASANAGVLLISGLRSEDEAD
YYCMIWHSSAAVFGGGTQLTVLTTTPAPRPPTPAPTIASQPLSLRPEACRPAA
GGAVHTRGLDFACDFWVLVVVGGVLACYSLLVTVAFIIFWVRSKRSRLLHSD
YMNMTPRRPGPTRKHYQPYAPPRDFAAYRSRVKFSRSADAPAYQQGQNQLY
NELNLGRREEYDVLDKRRGRDPEMGGKPRRKNPQEGLYNELQKDKMAEAY
SEIGMKGERRRGKGHDGLYQGLSTATKDTYDALHMQALPPREGRGSLLTCG
DVEENPGPMGRGLLRGLWPLHIVLWTRIASTIPPHVQKSDVEMEAQKDEIICP
SCNRTAHPLRHINNDMIVTDNNGAVKFPQLCKFCDVRFSTCDNQKSCMSNCS
ITSICEKPQEVCVAVWRKNDENITLETVCHDPKLPYHDFILEDAASPKCIMKE
KKKPGETFFMCSCSSDECNDNIIFSEEYNTSNPDLLLVIFQ
SEQ ID NO: 13 (amino acid sequence of MesoCAR06) 
MALPVTALLLPLALLLHAARPQVQLQQSGPGLVTPSQTLSLTCAISGDSV
SSNSATWNWIRQSPSRGLEWLGRTYYRSKWYNDYAVSVKSRMSINPDTSKN
QFSLQLNSVTPEDTAVYYCARGMMTYYYGMDVWGQGTTVTVSSGILGSGG
GGSGGGGSGGGGSQPVLTQSSSLSASPGASASLTCTLRSGINVGPYRIYWYQQ
KPGSPPQYLLNYKSDSDKQQGSGVPSRFSGSKDASANAGVLLISGLRSEDEAD
 
YYCMIWHSSAAVFGGGTQLTVLTTTPAPRPPTPAPTIASQPLSLRPEACRPAA
GGAVHTRGLDFACDFWVLVVVGGVLACYSLLVTVAFIIFWVRSKRSRLLHSD
YMNMTPRRPGPTRKHYQPYAPPRDFAAYRSRVKFSRSADAPAYQQGQNQLY
NELNLGRREEYDVLDKRRGRDPEMGGKPRRKNPQEGLYNELQKDKMAEAY
SEIGMKGERRRGKGHDGLYQGLSTATKDTYDALHMQALPPREGRGSLLTCG
DVEENPGPMGRGLLRGLWPLHIVLWTRIASTIPPHVQKSVNNDMIVTDNNGA
VKFPQLCKFCDVRFSTCDNQKSCMSNCSITSICEKPQEVCVAVWRKNDENITL
ETVCHDPKLPYHDFILEDAASPKCIMKEKKKPGETFFMCSCSSDECNDNIIFSE
EYNTSNPDLLLVIFQ
SEQ ID NO: 14 (amino acid sequence of MesoCAR07) 
MALPVTALLLPLALLLHAARPQVQLQQSGPGLVTPSQTLSLTCAISGDSV
SSNSATWNWIRQSPSRGLEWLGRTYYRSKWYNDYAVSVKSRMSINPDTSKN
QFSLQLNSVTPEDTAVYYCARGMMTYYYGMDVWGQGTTVTVSSGILGSGG
GGSGGGGSGGGGSQPVLTQSSSLSASPGASASLTCTLRSGINVGPYRIYWYQQ
KPGSPPQYLLNYKSDSDKQQGSGVPSRFSGSKDASANAGVLLISGLRSEDEAD
YYCMIWHSSAAVFGGGTQLTVLTTTPAPRPPTPAPTIASQPLSLRPEACRPAA
GGAVHTRGLDFACDFWVLVVVGGVLACYSLLVTVAFIIFWVRSKRSRLLHSD
YMNMTPRRPGPTRKHYQPYAPPRDFAAYRSRVKFSRSADAPAYQQGQNQLY
NELNLGRREEYDVLDKRRGRDPEMGGKPRRKNPQEGLYNELQKDKMAEAY
SEIGMKGERRRGKGHDGLYQGLSTATKDTYDALHMQALPPRATNFSLLKQA
GDVEENPGPMDWTWILFLVAAATRVHSNWVNVISDLKKIEDLIQSMHIDATL
YTESDVHPSCKVTAMKCFLLELQVISLESGDASIHDTVENLIILANDSLSSNGN
VTESGCKECEELEEKNIKEFLQSFVHIVQMFINTSSGGGSGGGGSGGGGSGGG
GSGGGSLQITCPPPMSVEHADIWVKSYSLYSRERYICNSGFKRKAGTSSLTEC
VLNKATNVAHWTTPSLKCIRDPALVHQRPAPPSTVTTAGVTPQPESLSPSGKE
PAASSPSSNNTAATTAAIVPGSQLMPSKSPSTGTTEISSHESSHGTPSQTTAKN
WELTASASHQPPGVYPQGHSDTTVAISTSTVLLCGLSAVSLLACYLKSRQTPP
LASVEMEAMEALPVTWGTSSRDEDLENCSHHLEGRGSLLTCGDVEENPGPM
GRGLLRGLWPLHIVLWTRIASTIPPHVQKSDVEMEAQKDEIICPSCNRTAHPLR
HINNDMIVTDNNGAVKFPQLCKFCDVRFSTCDNQKSCMSNCSITSICEKPQEV
CVAVWRKNDENITLETVCHDPKLPYHDFILEDAASPKCIMKEKKKPGETFFM
CSCSSDECNDNIIFSEEYNTSNPDLLLVIFQ
SEQ ID NO: 15 (amino acid sequence of MesoCAR08) 
MALPVTALLLPLALLLHAARPQVQLQQSGPGLVTPSQTLSLTCAISGDSV
SSNSATWNWIRQSPSRGLEWLGRTYYRSKWYNDYAVSVKSRMSINPDTSKN
QFSLQLNSVTPEDTAVYYCARGMMTYYYGMDVWGQGTTVTVSSGILGSGG
GGSGGGGSGGGGSQPVLTQSSSLSASPGASASLTCTLRSGINVGPYRIYWYQQ
KPGSPPQYLLNYKSDSDKQQGSGVPSRFSGSKDASANAGVLLISGLRSEDEAD
YYCMIWHSSAAVFGGGTQLTVLTTTPAPRPPTPAPTIASQPLSLRPEACRPAA
GGAVHTRGLDFACDFWVLVVVGGVLACYSLLVTVAFIIFWVRSKRSRLLHSD
YMNMTPRRPGPTRKHYQPYAPPRDFAAYRSRVKFSRSADAPAYQQGQNQLY
NELNLGRREEYDVLDKRRGRDPEMGGKPRRKNPQEGLYNELQKDKMAEAY
SEIGMKGERRRGKGHDGLYQGLSTATKDTYDALHMQALPPRATNFSLLKQA
GDVEENPGPMDWTWILFLVAAATRVHSNWVNVISDLKKIEDLIQSMHIDATL
YTESDVHPSCKVTAMKCFLLELQVISLESGDASIHDTVENLIILANDSLSSNGN
VTESGCKECEELEEKNIKEFLQSFVHIVQMFINTSSGGGSGGGGSGGGGSGGG
GSGGGSLQITCPPPMSVEHADIWVKSYSLYSRERYICNSGFKRKAGTSSLTEC
VLNKATNVAHWTTPSLKCIRDPALVHQRPAPPSTVTTAGVTPQPESLSPSGKE
PAASSPSSNNTAATTAAIVPGSQLMPSKSPSTGTTEISSHESSHGTPSQTTAKN
WELTASASHQPPGVYPQGHSDTTVAISTSTVLLCGLSAVSLLACYLKSRQTPP
LASVEMEAMEALPVTWGTSSRDEDLENCSHHLEGRGSLLTCGDVEENPGPGR
GLLRGLWPLHIVLWTRIASTIPPHVQKSVNNDMIVTDNNGAVKFPQLCKFCD
VRFSTCDNQKSCMSNCSITSICEKPQEVCVAVWRKNDENITLETVCHDPKLPY
HDFILEDAASPKCIMKEKKKPGETFFMCSCSSDECNDNIIFSEEYNTSNPDLLL
VIFQ
SEQ ID NO: 16 (amino acid sequence of MesoCAR09) 
MALPVTALLLPLALLLHAARPQVQLQQSGPGLVTPSQTLSLTCAISGDSV
SSNSATWNWIRQSPSRGLEWLGRTYYRSKWYNDYAVSVKSRMSINPDTSKN
QFSLQLNSVTPEDTAVYYCARGMMTYYYGMDVWGQGTTVTVSSGILGSGG
GGSGGGGSGGGGSQPVLTQSSSLSASPGASASLTCTLRSGINVGPYRIYWYQQ
KPGSPPQYLLNYKSDSDKQQGSGVPSRFSGSKDASANAGVLLISGLRSEDEAD
YYCMIWHSSAAVFGGGTQLTVLTTTPAPRPPTPAPTIASQPLSLRPEACRPAA
GGAVHTRGLDFACDFWVLVVVGGVLACYSLLVTVAFIIFWVRSKRSRLLHSD
 
YMNMTPRRPGPTRKHYQPYAPPRDFAAYRSRVKFSRSADAPAYQQGQNQLY
NELNLGRREEYDVLDKRRGRDPEMGGKPRRKNPQEGLYNELQKDKMAEAY
SEIGMKGERRRGKGHDGLYQGLSTATKDTYDALHMQALPPRATNFSLLKQA
GDVEENPGPMDWTWILFLVAAATRVHSNWVNVISDLKKIEDLIQSMHIDATL
YTESDVHPSCKVTAMKCFLLELQVISLESGDASIHDTVENLIILANDSLSSNGN
VTESGCKECEELEEKNIKEFLQSFVHIVQMFINTSSGGGSGGGGSGGGGSGGG
GSGGGSLQITCPPPMSVEHADIWVKSYSLYSRERYICNSGFKRKAGTSSLTEC
VLNKATNVAHWTTPSLKCIRDPALVHQRPAPPSTVTTAGVTPQPESLSPSGKE
PAASSPSSNNTAATTAAIVPGSQLMPSKSPSTGTTEISSHESSHGTPSQTTAKN
WELTASASHQPPGVYPQGHSDTTVAISTSTVLLCGLSAVSLLACYLKSRQTPP
LASVEMEAMEALPVTWGTSSRDEDLENCSHHLEGRGSLLTCGDVEENPGPM
GRGLLRGLWPLHIVLWTRIASTIPPHVQKSDVEMEAQKDEIICPSCNRTAHPLR
HINNDMIVTDNNGAVKFPQLCKFCDVRFSTCDNQKSCMSNCSITSICEKPQEV
CVAVWRKNDENITLETVCHDPKLPYHDFILEDAASPKCIMKEKKKPGETFFM
CSCSSDECNDNIIFSEEYNTSNPDLLLVIFQPILLTISILSFFSVALLVILACVLWK
KRIKPIVWPSLPDHKKTLEHLCKKPRKNLNVSFNPESFLDCQIHRVDDIQARD
EVEGFLQDTFPQQLEESEKQRLGGDVQSPNCPSEDVVITPESFGRDSSLTCLAG
NVSACDAPILSSSRSLDCRESGKNGPHVYQDLLLSLGTTNSTLPPPFSLQSGILT
LNPVAQGQPILTSLGSNQEEAYVTMSSFYQNQ
SEQ ID NO: 17 (amino acid sequence of MesoCAR10) 
MALPVTALLLPLALLLHAARPQVQLQQSGPGLVTPSQTLSLTCAISGDSV
SSNSATWNWIRQSPSRGLEWLGRTYYRSKWYNDYAVSVKSRMSINPDTSKN
QFSLQLNSVTPEDTAVYYCARGMMTYYYGMDVWGQGTTVTVSSGILGSGG
GGSGGGGSGGGGSQPVLTQSSSLSASPGASASLTCTLRSGINVGPYRIYWYQQ
KPGSPPQYLLNYKSDSDKQQGSGVPSRFSGSKDASANAGVLLISGLRSEDEAD
YYCMIWHSSAAVFGGGTQLTVLTTTPAPRPPTPAPTIASQPLSLRPEACRPAA
GGAVHTRGLDFACDFWVLVVVGGVLACYSLLVTVAFIIFWVRSKRSRLLHSD
YMNMTPRRPGPTRKHYQPYAPPRDFAAYRSRVKFSRSADAPAYQQGQNQLY
NELNLGRREEYDVLDKRRGRDPEMGGKPRRKNPQEGLYNELQKDKMAEAY
SEIGMKGERRRGKGHDGLYQGLSTATKDTYDALHMQALPPRATNFSLLKQA
GDVEENPGPMDWTWILFLVAAATRVHSNWVNVISDLKKIEDLIQSMHIDATL
YTESDVHPSCKVTAMKCFLLELQVISLESGDASIHDTVENLIILANDSLSSNGN
 
VTESGCKECEELEEKNIKEFLQSFVHIVQMFINTSSGGGSGGGGSGGGGSGGG
GSGGGSLQITCPPPMSVEHADIWVKSYSLYSRERYICNSGFKRKAGTSSLTEC
VLNKATNVAHWTTPSLKCIRDPALVHQRPAPPSTVTTAGVTPQPESLSPSGKE
PAASSPSSNNTAATTAAIVPGSQLMPSKSPSTGTTEISSHESSHGTPSQTTAKN
WELTASASHQPPGVYPQGHSDTTVAISTSTVLLCGLSAVSLLACYLKSRQTPP
LASVEMEAMEALPVTWGTSSRDEDLENCSHHLEGRGSLLTCGDVEENPGPM
GRGLLRGLWPLHIVLWTRIASTIPPHVQKSDVEMEAQKDEIICPSCNRTAHPLR
HINNDMIVTDNNGAVKFPQLCKFCDVRFSTCDNQKSCMSNCSITSICEKPQEV
CVAVWRKNDENITLETVCHDPKLPYHDFILEDAASPKCIMKEKKKPGETFFM
CSCSSDECNDNIIFSEEYNTSNPDLLLVIFQPILLTCPTISILSFFSVALLVILACVL
WKKRIKPIVWPSLPDHKKTLEHLCKKPRKNLNVSFNPESFLDCQIHRVDDIQA
RDEVEGFLQDTFPQQLEESEKQRLGGDVQSPNCPSEDVVITPESFGRDSSLTCL
AGNVSACDAPILSSSRSLDCRESGKNGPHVYQDLLLSLGTTNSTLPPPFSLQSG
ILTLNPVAQGQPILTSLGSNQEEAYVTMSSFYQNQ
SEQ ID NO: 18 (amino acid sequence of E1m1) 
MALPVTALLLPLALLLHAARPQVQLQQSGPGLVTPSQTLSLTCAISGDSV
SSNSATWNWIRQSPSRGLEWLGRTYYRSKWYNDYAVSVKSRMSINPDTSKN
QFSLQLNSVTPEDTAVYYCARGMMTYYYGMDVWGQGTTVTVSSGILGSGG
GGSGGGGSGGGGSQPVLTQSSSLSASPGASASLTCTLRSGINVGPYRIYWYQQ
KPGSPPQYLLNYKSDSDKQQGSGVPSRFSGSKDASANAGVLLISGLRSEDEAD
YYCMIWHSSAAVFGGGTQLTVLTTTPAPRPPTPAPTIASQPLSLRPEACRPAA
GGAVHTRGLDFACDFWVLVVVGGVLACYSLLVTVAFIIFWVRSKRSRLLHSD
YMNMTPRRPGPTRKHYQPYAPPRDFAAYRSRVKFSRSADAPAYQQGQNQLY
NELNLGRREEYDVLDKRRGRDPEMGGKPRRKNPQEGLYNELQKDKMAEAY
SEIGMKGERRRGKGHDGLYQGLSTATKDTYDALHMQALPPRGSGATNFSLL
KQAGDVEENPGPMDWTWILFLVAAATRVHSNWVNVISDLKKIEDLIQSMHID
ATLYTESDVHPSCKVTAMKCFLLELQVISLESGDASIHDTVENLIILANDSLSS
NGNVTESGCKECEELEEKNIKEFLQSFVHIVQMFINTSSGGGSGGGGSGGGGS
GGGGSGGGSLQITCPPPMSVEHADIWVKSYSLYSRERYICNSGFKRKAGTSSL
TECVLNKATNVAHWTTPSLKCIRDPALVHQRPAPPSTVTTAGVTPQPESLSPS
GKEPAASSPSSNNTAATTAAIVPGSQLMPSKSPSTGTTEISSHESSHGTPSQTTA
KNWELTASASHQPPGVYPQGHSDTTVAISTSTVLLCGLSAVSLLACYLKSRQT
 PPLASVEMEAMEALPVTWGTSSRDEDLENCSHHL
SEQ ID NO: 19 (amino acid sequence of CAR11) 
MGRGLLRGLWPLHIVLWTRIASTIPPHVQKSDVEMEAQKDEIICPSCNRT
AHPLRHINNDMIVTDNNGAVKFPQLCKFCDVRFSTCDNQKSCMSNCSITSICE
KPQEVCVAVWRKNDENITLETVCHDPKLPYHDFILEDAASPKCIMKEKKKPG
ETFFMCSCSSDECNDNIIFSEECKPDHGYWSAWSPATFIQIPSDFTMNDTTVWI
SVAVLSAVICLIIVWAVALKKRIKPIVWPSLPDHKKTLEHLCKKPRKNLNVSF
NPESFLDCQIHRVDDIQARDEVEGFLQDTFPQQLEESEKQRLGGDVQSPNCPS
EDVVITPESFGRDSSLTCLAGNVSACDAPILSSSRSLDCRESGKNGPHVYQDLL
LSLGTTNSTLPPPFSLQSGILTLNPVAQGQPILTSLGSNQEEAYVTMSSFYQNQ
SEQ ID NO: 20 (amino acid sequence of CAR12) 
MGRGLLRGLWPLHIVLWTRIASTIPPHVQKSDVEMEAQKDEIICPSCNRT
AHPLRHINNDMIVTDNNGAVKFPQLCKFCDVRFSTCDNQKSCMSNCSITSICE
KPQEVCVAVWRKNDENITLETVCHDPKLPYHDFILEDAASPKCIMKEKKKPG
ETFFMCSCSSDECNDNIIFSEEKVRSIPDHYFKGFWSEWSPSYYFRTPEINNSSG
EMDPILLTISILSFFSVALLVILACVLWKKRIKPIVWPSLPDHKKTLEHLCKKPR
KNLNVSFNPESFLDCQIHRVDDIQARDEVEGFLQDTFPQQLEESEKQRLGGDV
QSPNCPSEDVVITPESFGRDSSLTCLAGNVSACDAPILSSSRSLDCRESGKNGP
HVYQDLLLSLGTTNSTLPPPFSLQSGILTLNPVAQGQPILTSLGSNQEEAYVTM
SSFYQNQ
SEQ ID NO: 21 (amino acid sequence of CAR13) 
MALPVTALLLPLALLLHAARPQLCKFCDVRFSTCDNQKSCMSNCSITSIC
EKPQEVCVAVWRKNDENITLETVCHDPKLPYHDFILEDAASPKCIMKEKKKP
GETFFMCSCSSDECNDNIIFSEEYNTSNPDLLLVIFQSDHLVLGLSAVLGLLLLR
WQFPAHYRRLRHALWPSLPDLHRVLGQYLRDTAALSPPKATVSDTCEEVEPS
LLEILPKSSERTPLPLLEARDEVEGFLQDTFPQQLEESEKQRLGGDVQSPNCPS
EDVVITPESFGRDSSLTCLAGNVSACDAPILSSSRSLDCRESGKNGPHVYQDLL
LSLGTTNSTLPPPFSLQSGILTLNPVAQGQPILTSLGSNQEEAYVTMSSFYQNQ
SRSDPKPENPACPWTVLPAGDLPTHDGYLPSNIDDLPSHEAPLADSLEELEPQ
SEQ ID NO: 22 (TM2 element: selected from the transmembrane region of PRLR) 
CKPDHGYWSAWSPATFIQIPSDFTMNDTTVWISVAVLSAVICLIIVWAVA
L
SEQ ID NO: 23 (TM2 element: selected from part of the extracellular region and the transmembrane region of IL-7R) 
KVRSIPDHYFKGFWSEWSPSYYFRTPEINNSSGEMDPILLTISILSFFSVAL
LVILACVLW
SEQ ID NO: 24 (TM2 element: selected from part of the transmembrane region of TPOR)
   SDHLVLGLSAVLGLLLL

### Example 1 Isolation of PBMC from donor blood and expansion of T cells

Monocytes were isolated from peripheral blood, density gradient centrifugation was performed using Ficoll, and T cells were enriched (Pan T kit, Miltenyi). Then T cells were activated by magnetic beads coupled Anti-CD3/anti-CD28, cultured and expanded. The cell culture medium x-vivo 15 (with 5% FBS, 300 IU / mL rhIL-2) was used, and the cells were cultured continuously at 37°C in a 5% CO₂ incubator.

### Example 2 Cell culture and construction

Cell lines expressing MSLN: OVCAR3 (human ovarian cancer cell line, ATCC HTB-161), HCC70 (human breast cancer cell line). The culture medium was prepared according to ATCC guidelines.

### Example 3 CAR structure design and transduction

The present invention constructs the fourth generation Meso-CAR molecules based on the second generation Meso-CAR molecule. Meso-CAR is a CAR structure targeting mesothelin with a basic structure including a signal peptide, P4 scFV (specifically targeting human mesothelin), CD8 hinge region, CD28 transmembrane region, and CD28 costimulatory signaling domain. Using different intracellular costimulatory signals, a total of 10 Meso-CARs were constructed, and their specific structural compositions are shown in Table 2.

**Table 2**

| Name | Same structural parts | Signal conversion protein | IL-15m1/IL-15Ra |
|---|---|---|---|
| MesoCAR | SP+P4scFv+CD8H+28T MD+28CSD+CD3ζ | Absent | Not expressing |
| E1m1 | SP+P4scFv+CD8H+28T MD+28CSD+CD3ζ | Absent | Expressing |
| MesoCAR 01 | SP+P4scFv+CD8H+28T MD+28CSD+CD3ζ | Expressing Sc1-type (TGFBR2 isoA and IL-7Rα transmembrane region and intracellular region) | Not expressing |
| MesoCAR 02 | SP+P4scFv+CD8H+28T MD+28CSD+CD3ζ | Expressing Sc2-type (TGFBR2 isoB and IL-7Rα transmembrane region and intracellular region) | Not expressing |
| MesoCAR 03 | SP+P4scFv+CD8H+28T MD+28CSD+CD3ζ | Expressing Sc3-type (TGFBR2 isoA and IL-7Rα transmembrane region mutant and intracellular region) | Not expressing |
| MesoCAR 04 | SP+P4scFv+CD8H+28T MD+28CSD+CD3ζ | Expressing Sc4-type (TGFBR2 isoB and IL-7Rα transmembrane region mutant and intracellular region) | Not expressing |
| MesoCAR 05 | SP+P4scFv+CD8H+28T MD+28CSD+CD3ζ | Only containing TGFBR2 SP and TGFBR2 isoA, without IL-7Rα transmembrane region, IL-7Rα transmembrane region mutant or intracellular region | Not expressing |
| MesoCAR 06 | SP+P4scFv+CD8H+28T MD+28CSD+CD3ζ | Only containing TGFBR2 SP and TGFBR2 isoB, without IL-7Rα transmembrane region, IL-7Rα transmembrane region mutant or intracellular region | Not expressing |
| MesoCAR 07 | SP+P4scFv+CD8H+28T MD+28CSD+CD3ζ | Only containing TGFBR2 SP and TGFBR2 isoA, without IL-7Rα transmembrane region, IL-7Rα transmembrane region mutant or intracellular region, and only containing IL-15m1/IL-15Ra | Expressing |
| MesoCAR 08 | SP+P4scFv+CD8H+28T MD+28CSD+CD3ζ | Only containing TGFBR2 SP and TGFBR2 isoB, without IL-7Rα transmembrane region, IL-7Rα transmembrane region mutant or intracellular region, and only containing IL-15m1/IL-15Ra | Expressing |
| MesoCAR 09 | SP+P4scFv+CD8H+28T MD+28CSD+CD3ζ | Expressing Sc1-type (TGFBR2 isoA, IL-7Rα transmembrane region and intracellular region, and IL-15m1/IL-15R) | Expressing |
| MesoCAR 10 | SP+P4scFv+CD8H+28T MD+28CSD+CD3ζ | Expressing Sc3-type (TGFBR2 isoA, IL-7Rα transmembrane region mutant and intracellular region, and IL-15m1/IL-15R) | Expressing |
| CAR11 | SP+VHH+CD8H+28TM D+28CSD+CD3ζ | TGFBR2 isoA and PRTR transmembrane region and IL-7Rα intracellular region | Not expressing |
| CAR12 | SP+VHH+CD8H+28TM D+28CSD+CD3ζ | TGFBR2 isoA and IL-7Rα transmembrane region (including WSXWS) and IL-7Rα intracellular region | Not expressing |
| CAR13 | SP+VHH+CD8H+28TM D+28CSD+CD3ζ | TGFBR2 isoB and TPOR transmembrane region and IL-7Rα intracellular region | Not expressing |

Among them, the specific structures of the CAR molecules from their amino terminus to carboxyl terminus are as follows:
MESOCAR consists of SP, P4scFv, CD8H, 28TMD, 28CSD and CD3ζ in series connection.

E1m1 consists of SP, P4scFv, CD8H, 28TMD, 28CSD, CD3ζ, P2A, IL-15m1 / IL-15Ra SP: IgE, IL-15m1, Linker and IL-15Ra in series connection.

MesoCAR01 consists of SP, P4scFv, CD8H, 28TMD, 28CSD, CD3ζ, T2A and Sc1-type in series connection.

MesoCAR02 consists of SP, P4scFv, CD8H, 28TMD, 28CSD, CD3ζ, T2A and Sc2-type in series connection.

MesoCAR03 consists of SP, P4scFv, CD8H, 28TMD, 28CSD, CD3ζ, T2A and Sc3-type in series connection.

MesoCAR04 consists of SP, P4scFv, CD8H, 28TMD, 28CSD, CD3ζ, T2A and Sc4-type in series connection.

MesoCAR05 consists of SP, P4scFv, CD8H, 28TMD, 28CSD, CD3ζ, T2A, TGFBR2 SP and TGFBR2 isoAin series connection.

MesoCAR06 consists of SP, P4scFv, CD8H, 28TMD, 28CSD, CD3ζ, T2A, TGFBR2 SP and TGFBR2 isoB in series connection.

MesoCAR07 consists of SP, P4scFv, CD8H, 28TMD, 28CSD, CD3ζ, T2A, TGFBR2 SP, TGFBR2 isoA, P2A, IL-15m1/IL-15Ra SP: IgE, IL-15m1, Linker and IL-15Ra in series connection.

MesoCAR08 consists of SP, P4scFv, CD8H, 28TMD, 28CSD, CD3ζ, T2A, TGFBR2 SP, TGFBR2 isoB, T2A, IL-15m1/IL-15Ra SP: IgE, IL-15m1, Linker and IL-15Ra in series connection.

MesoCAR09 consists of SP, P4scFv, CD8H, 28TMD, 28CSD, CD3ζ, P2A, IL-15m1/IL-15Ra SP: IgE, IL-15m1, Linker, IL-15Ra, T2A and Sc1-type in series connection.

MesoCAR10 consists of SP, P4scFv, CD8H, 28TMD, 28CSD, CD3ζ, P2A, IL-15m1/IL-15Ra SP: IgE, IL-15m1, Linker, IL-15Ra, T2A and Sc3-type in series connection.

CAR11 consists of SP, VHH, CD8H, 28TMD, 28CSD, CD3ζ, T2A/P2A, TGFBR2 SP, TGFBR2 isoA, PRTR transmembrane region and IL-7R intracellular region in series connection.

CAR12 consists of SP, VHH, CD8H, 28TMD, 28CSD, CD3ζ, T2A/P2A, TGFBR2 SP, TGFBR2 isoA, IL-7Rα transmembrane region (including WSXWS) and IL-7R intracellular region in series connection.

CAR13 consists of SP, VHH, CD8H, 28TMD, 28CSD, CD3ζ, T2A/P2A, TGFBR2 SP, TGFBR2 isoB, TPOR transmembrane region and IL-7Rα intracellular regions in series connection.

Among them, the self-cleaving 2A peptide P2A and T2A can be used interchangeably.

The Meso-CAR genes listed above were cloned into the FUW lentiviral vector and transfected into 293T cells using PEI transfection reagents along with the lentiviral packaging plasmids pMD2.G (Addgene, Plasmid # 12259) and psPAX2 (Addgene, Plasmid # 12260). The vectors were expressed and the viruses were collected at 48 hours and 72 hours, respectively. After ultracentrifugation and concentration, T cells were infected and activated.

### Example 4 CAR-T cell preparation - lentiviral infection, detection of CAR positive ratio, and in vitro proliferation

After 48 hours of activation, primary T cells were added with the concentrated lentivirus in Example 3. After 72 hours of cell infection, the CAR positive ratio was detected using MSLN antigen, TGFBR2, and IL-15R antibody, respectively.

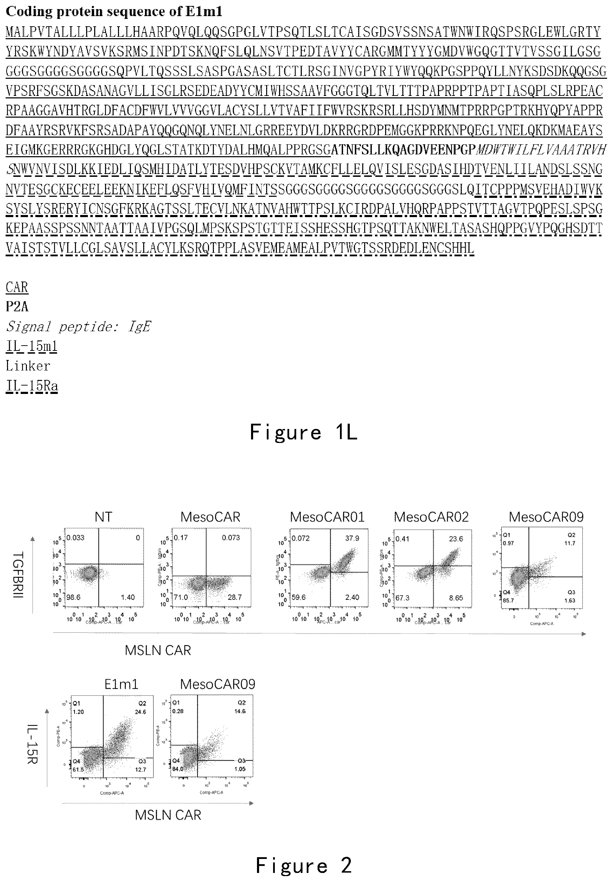

The flow cytometry results were shown in Figure 2. The CAR expression of all four CAR-T cells was above 10%. TGFBR2 and MSLN CAR were simultaneously expressed on the surfaces of MesoCAR01, MesoCAR02, and MesoCAR10 CAR-cells, wherein on the surface of MesoCAR10 CAR-cells, TGFBR2, IL-15R and MSLN CAR were simultaneously expressed.

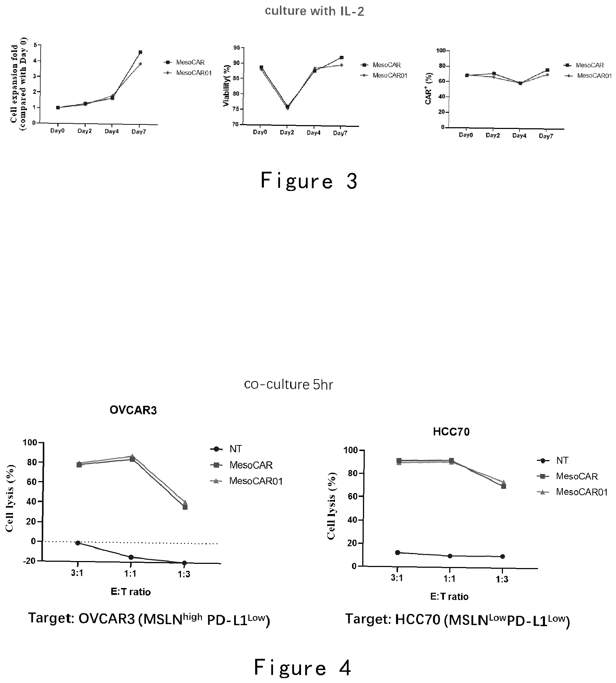

After transfection, T cells were continuously cultured in a 5% CO₂ incubator at 37°C, using x-vivo 15 + 300 IU IL-2 + 5% FBS as the culture medium. The culture medium was supplemented once every other day, and the cells were harvested on Day 10. After cell cryopreservation, the cells were resuscitated and continued to be cultured. CAR positive rate, survival ratio, and count were measured every 2-3 days to calculate the proliferation rate of CAR positive cells. The experimental results were shown in Figure 3. After cell resuscitation from Day 0 to Day 7, there was no significant difference in CAR-T cell expansion, cell viability, and CAR positive ratio between MESOCAR and MesoCAR01 expressing the signal conversion protein.

### Example 5 In vitro cell killing

*In vitro* killing experiments were performed on the T cells harvested in Example 4. By using the RTCA method, CAR-T cells were co-incubated with target cells at a ratio of 3:1, 1:1, and 1:3 for 1-2 consecutive days. The growth status of the target cells was recorded in real-time, and the cell survival rate was measured to obtain the killing efficiency of the CAR-T cells.

As shown in Figure 4, when MesoCAR01 and MESOCAR CAR-T cells were co-cultured with tumor cells OVCAR3 or HCC70 (E:T = 3:1, 1:1, 1:3), there was no significant difference in killing activity. When non-transduced T cells (NT) were co-cultured with tumor cells OVCAR3 or HCC70 (E:T = 3:1, 1:1, 1:3), there was no significant killing effect on the tumor cells.

### Example 6 pSTATS

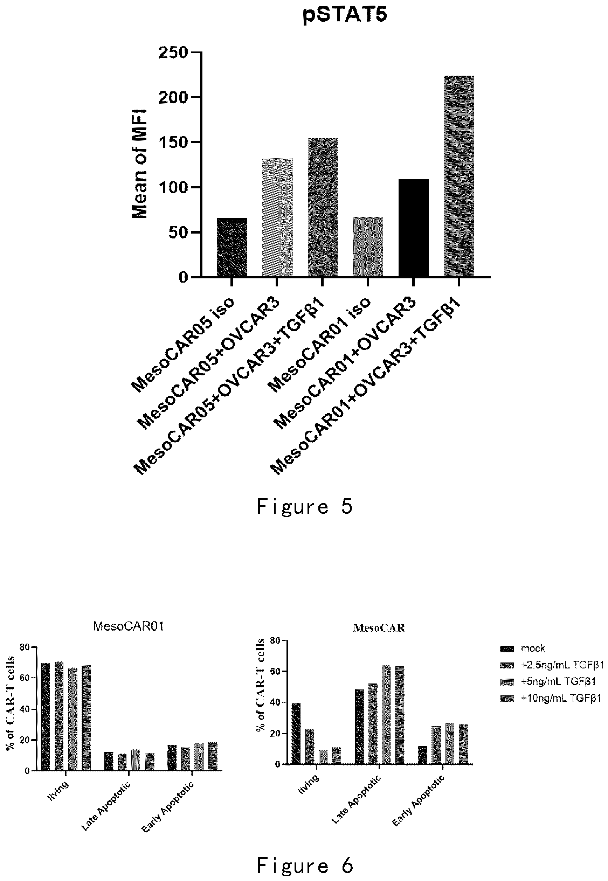

*In vitro* killing experiments were performed on the T cells harvested in Example 4. CAR-T cells were co-incubated with target cells OVCAR3 at a ratio of E:T = 1:1 for 3 hours, and the co-incubated cells were divided into two groups during the culture process, i.e., the group with TGF-β1 / the group without TGF-β1, respectively. The expression of pSTAT5 in each group of cells was detected by FACS, and Mean MFI values thereof were counted. As shown in Figure 5, in the presence of TGF-β, the expression of pSTAT5 in MesoCAR01 was significantly upregulated, while there was no significant change in the expression of pSTAT5 in MesoCAR05. These results indicated that after treating MesoCAR01 CAR-T with TGF-β for 0.5 hr, the pSTAT5 signaling pathway was significantly activated, while the pSTAT5 signaling pathway of MesoCAR05 was not activated due to its lack of intracellular cytokine signaling expression.

### Example 7 Annexin V staining

*In vitro* killing experiments were performed on the T cells harvested in Example 4. CAR-T cells were co-incubated with target cells (MDA-MB231) at a ratio of 1:2 for 3 consecutive days, and the co-incubated cells were divided into two groups during the culture process, i.e., the group with TGF-β1 / the group without TGF-β1, respectively. The apoptosis of the CAR-T cells was detected. As shown in Figure 6, MesoCAR01 CAR-T had the highest proportion of live cells, which were double-negative cells for Annexin V and 7-AAD, at 80%. Even when added with different concentrations of TGF-β, there was no significant change in the survival rate thereof. While the apoptotic cells induced by TGF-β1 in the MESOCAR group increased significantly with the increase of TGF-β concentration.

### Example 8 In vitro pharmacological study - multi-round killing

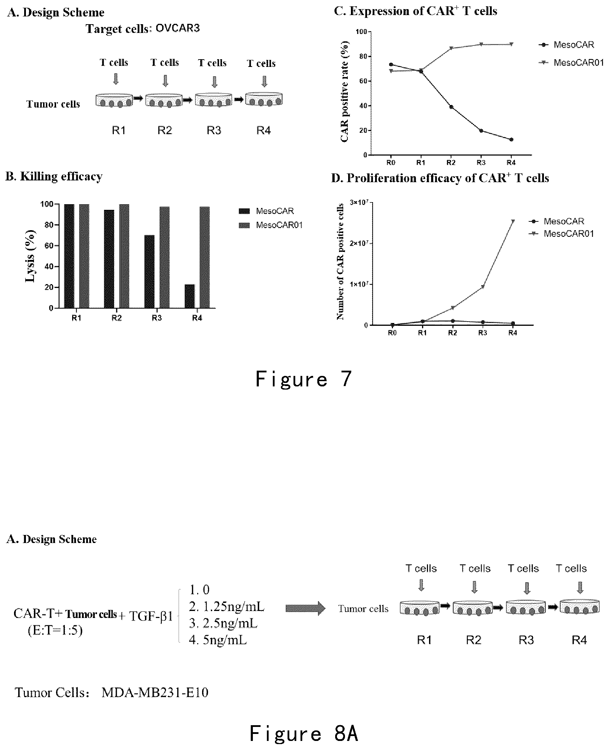

*In vitro* multi-round killing experiments were performed on the T cells harvested in Example 4. The experimental method was as shown in the schematic diagram (Figure 7A). CAR-T cells and target cells were co-incubated at a ratio of 1:3 for three consecutive days. The growth status of the target cells was observed under the microscope. On the fourth day of co-incubation, half of the cells were taken to detect the phenotype and number of T cells. The target cells were digested by trypsin, then the killing efficiency of the CAR-T cells was counted as the killing efficiency = (control group - experimental group) / control group* 100%. The remaining half of the cells continued to be co-cultured with new target cells, and several rounds of continuous killing were performed using the above method.

As shown in Figure 7B, under co-culture of CAR-T in each group with OVCAR3 (E:T = 1:3), MesoCAR01 showed a significant killing advantage after the third round of continuous culture. As shown in Figures 7C and 7D, a stable CAR⁺ T cell positive rate and sustained cell expansion rate were observed in MesoCAR01.

### Example 9 In vitro pharmacological study - multi-round killing in the presence of TGF-β

*In vitro* multi-round killing experiments were performed on the T cells harvested in Example 4. The experimental method was as shown in Figure 8A. CAR-T cells and target cells were co-incubated at a ratio of 1:5 for three consecutive days, and during the culture process, different concentrations of recombinant TGF-β were added and supplemented every two days. The growth status of the target cells was recorded in real-time. On the fourth day of co-incubation, half of the cells were taken to detect the phenotype and number of T cells. The target cells were digested by trypsin, then the killing efficiency of the CAR-T cells was counted as the killing efficiency = (control group - experimental group) / control group* 100%. The remaining half of the cells continued to be co-cultured with target cells, and several rounds of continuous killing were performed using the above method.

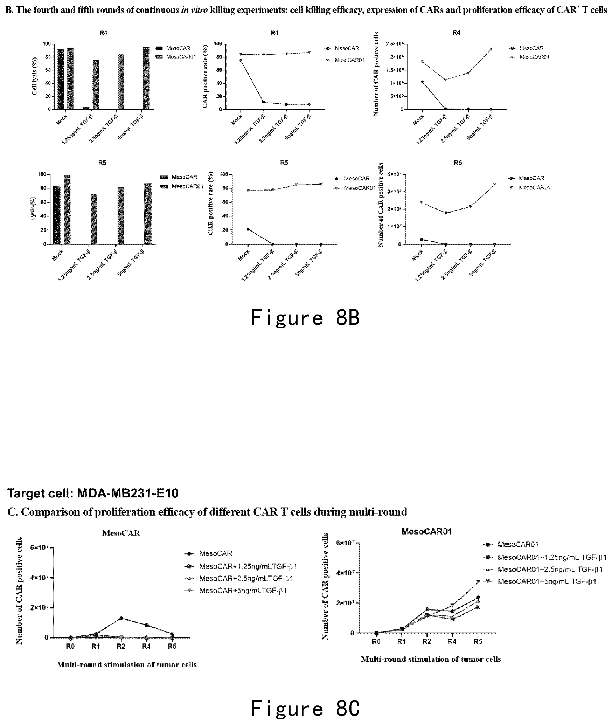

As shown in Figure 8B, under co-culture of CAR-T in each group with MDA-MB231-MSLN-E10 (a cell line with high expression of MSLN, constructed by transfecting MSLN-expressing plasmids into MDA-MB231 cells) (E:T=1:5), MesoCAR01 showed a significant killing advantage after the fourth round (R4) and the fifth round (R5) of continuous culture. Figures 8B and 8C showed stable CAR positive rates and cell expansion rates.

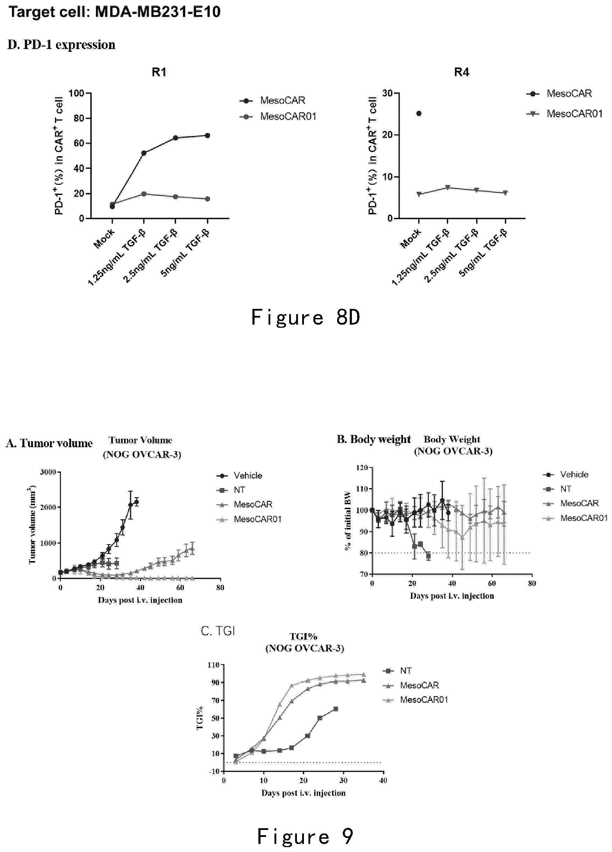

As shown in Figure 8D, after the first and fourth rounds of continuous killing, the expression of PD-1 in MesoCAR01 was significantly lower than that of MESOCAR, indicating that the immunosuppressive effects that were not conducive to tumor treatment were further reduced.

### Example 10 In vivo pharmacological study

NOD mice were selected, and subcutaneously injected with 5E6 OVCAR3 cells. The tumor load was continuously tested, and when the tumor was growing rapidly, the mice were divided into groups with 2-3 mice in each group. One day after grouping, 200 µL DPB S / mouse and 3E6 CAR-T / mouse were injected into the tail vein. On Day 1 after CAR-T injection, a small amount of mouse blood was taken to test the *in vivo* survival number of CAR-T cells. Afterwards, blood samples were taken once a week to test various phenotypes of CAR-T cells, and subcutaneous tumor sizes were measured twice a week.

As shown in Figure 9A, compared with the control group, the tumor load of mice injected with MESOCAR and MesoCAR01 cells was significantly reduced. Among them, the MESOCAR group mice showed an increase in tumor volume 20 days after CAR-T injection, while the MesoCAR01 group did not relapse.

As shown in Figure 9B, there was no significant change in the body weight of the animals in the MESOCAR and MesoCAR01 groups after CAR-T injection.

Figure 9C showed the tumor growth inhibition rates, wherein the tumor inhibition rates of mice inj ected with MESOCAR and MesoCAR01 cells were significantly higher than that of the control group (NT, Vehicle).

### Example 11 Detection of TGFβ/Smad signaling pathway

The CAR expression plasmid and two reporter gene vectors pNL (NLucP/SBE-RE/Hygro) (with a TGFβ signaling pathway regulatory element) and pGL4.51 [luc2/CMV/Neo] (reference reporter gene) were co-transfected into 293T cells.

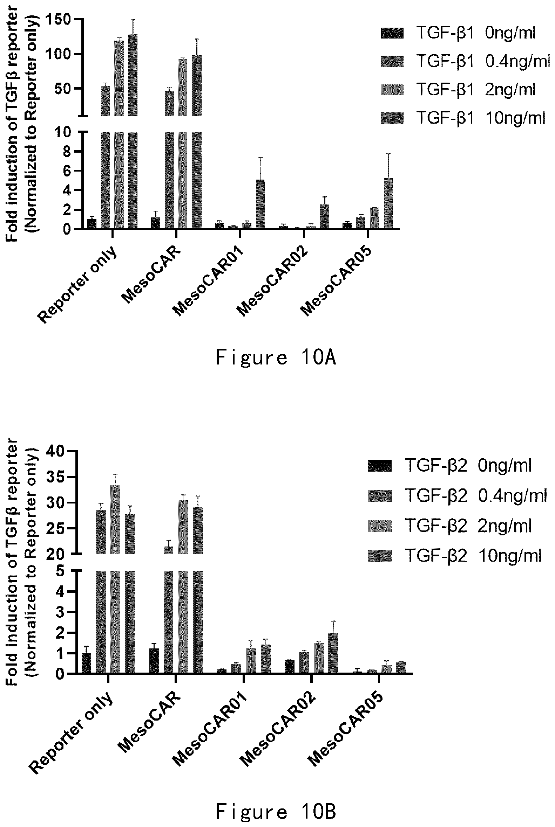

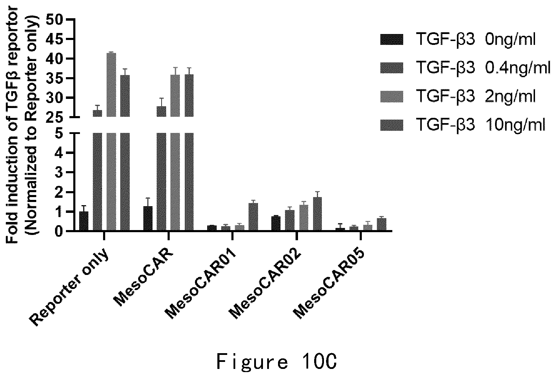

As shown in Figures 10A-10C, different concentrations of TGF-β1, TGF-β2, and TGF-β3 were added respectively after 24 hours, and incubated for another 20 hours, then the cells were added to Nano-Glo^{®} Dual-Luciferase^{®} Reporter Assay System for detection. Optical signal values were obtained and the inhibitory effects of two different DNTGFBR2 isoforms on TGFβ downstream signals were compared.

The experimental results showed that under different concentrations of TGF-β1, TGF-β2, and TGF-β3 treatments, MesoCAR01 and MesoCAR02 with the same intracellular conversion protein and different extracellular TGFBR2 receptor isoforms, both had significant inhibitory effects on the TGFβ/Smad signaling pathway. Among them, under low concentration of TGF-β3 (0.4 - 2 ng/ml) treatment, MesoCAR01 showed a better inhibitory effect on the TGFβ/Smad signaling pathway than MesoCAR02.

### Example 12 Detection of TGFβ/Smad and STATS signaling pathways

The CAR expression plasmid and two reporter gene vectors pNL (NLucP/SBE-RE/Hygro) (with a TGFβ signaling pathway regulatory element) or pNL (NLucP/STAT5-RE/Hygro), and pGL4.51 [luc2/CMV/Neo] (reference reporter gene) were co-transfected into 293T cells.

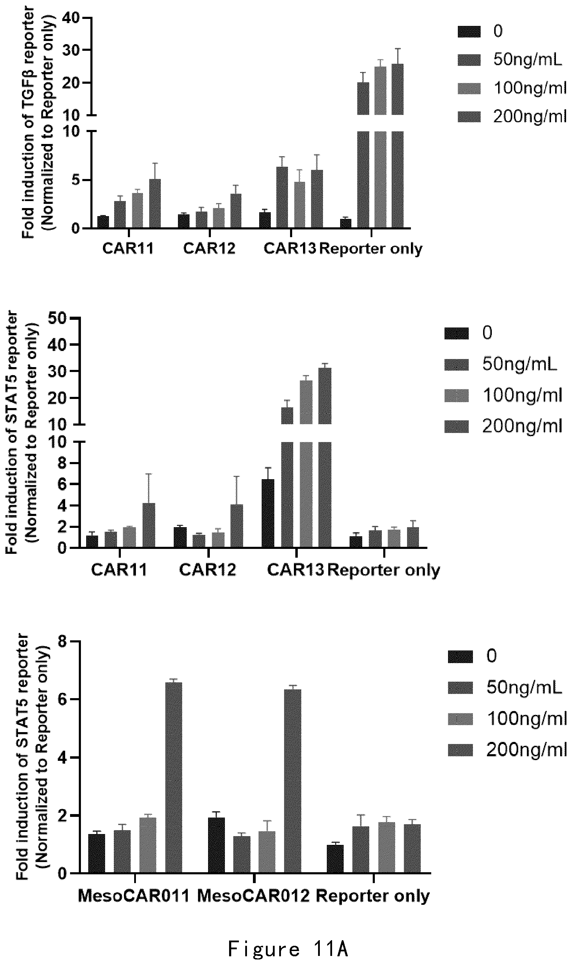

As shown in Figures 11A-11B, different concentrations of TGF-β1 were added respectively after 24 hours, and incubated for another 5 hours, then the cells were added to Nano-Glo^{®} Dual-Luciferase^{®} Reporter Assay System for detection. Optical signal values were obtained and the effects of DNTGFBR2 with different transmembrane sequences on TGFβ/STAT5 downstream signals were compared.

The experimental results showed that, as shown in Figure 11A, under the condition of adding with high concentrations of TGF-β1, CAR11, CAR12 and CAR13 all had a significant inhibitory effect on the TGFβ/Smad signaling pathway, while activating the STATS signaling pathway. Among them, CAR13 still had an activating effect on the STATS signaling pathway without the addition of TGFβ.

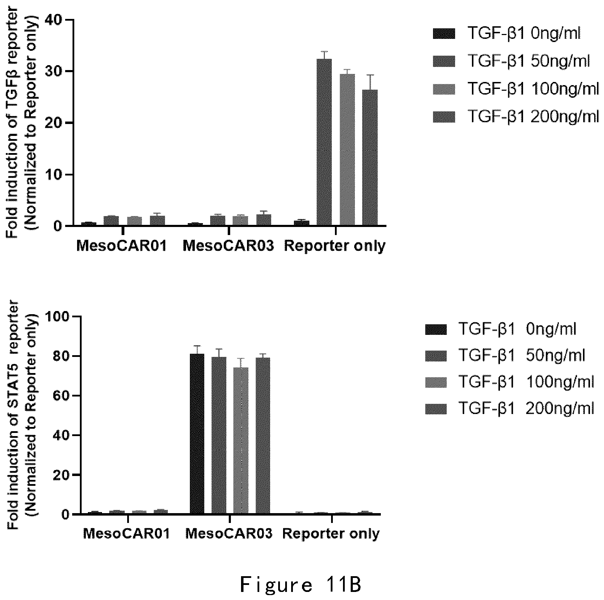

As shown in Figure 11B, when high concentrations of TGF-β1 were added, MesoCAR01 and MesoCAR03 both had a significant inhibitory effect on the TGFβ/Smad signaling pathway, and MesoCAR03 had a sustained activating effect on the STATS signaling pathway, even without the addition of TGF-β1.

### Discussion

The present invention constructed engineered immune cells based on targeted tumor antigens (such as human mesothelin, or claudin18.2). The engineered immune cells further expressed a fusion protein formed by the fusion of TGFBR2 and IL-7Rα. The fusion protein can act as a signal conversion protein, to block immunosuppressive signals in the tumor microenvironment and convert them into activation signals, thereby significantly and unexpectedly enhancing immune cell survival and tumor killing effects. In the present invention, the effects of fusion proteins connected by different transmembrane regions were also compared, and it was found that changes in the source of transmembrane regions did not affect the aforementioned effects of fusion proteins and engineered immune cells expressing corresponding fusion proteins.

In the present invention, immune cells having CARs for targeting tumor antigens and signal conversion proteins (such as a fusion protein formed by TGFBR2 and IL-7Rα) of the present invention can convert the inhibitory signals of TGF-β (including TGF-β inhibition of T cell proliferation, induction of cell apoptosis, and inhibition of inflammatory cytokine production, etc.) on T cells into activation signals, so as to prolong the *in vivo* survival time of CAR immune cells (such as CAR-T cells), thereby resisting the tumor microenvironment, effectively and continuously killing tumor cells, and reducing tumor recurrence.

All literatures mentioned in the present application are incorporated herein by reference, as though each one is individually incorporated by reference. In addition, it should be understood that, after reading the above teachings of the present invention, those skilled in the art can make various changes or modifications to the present invention, and all these equivalents also fall within the scope of claims as defined in the appended claims of the present application.

## Claims

1. An engineered immune cell, wherein the engineered immune cell is a T cell or NK cell, and the immune cell has the following characteristics:
(a) the immune cell expresses a chimeric antigen receptor (CAR), wherein the CAR targets surface markers of tumor cells; and
(b) the immune cell expresses an exogenous signal conversion protein, which is located on the cell membrane of the immune cell and has an extracellular domain of an immunosuppressive receptor, a transmembrane region (or a transmembrane domain), and an intracellular domain of an immunostimulatory receptor;
preferably, the immunosuppressive receptor is a TGF-β receptor;
preferably, the immunostimulatory receptor is selected from the group consisting of: IL-7 receptor, IL-15 receptor, IL-12 receptor, IL-2 receptor, IL-18 receptor, IL-21 receptor, and a combination thereof; more preferably, the immunostimulatory receptor is an IL-7 receptor.

2. The engineered immune cell of claim 1, wherein the CAR has a structure as shown in formula Ia or Ib:
L1-scFv-H-TM1-C-CD3ζ (Ia)
L1-scFv-H-TM1-C-CD3ζ-(A-S)n1-A-E-(A-S)n2 (Ib)
wherein,
L1 is absent or a signal peptide sequence;
scFv is a single chain variable domain of targeting antibodies;
H is absent or a hinge region;
TM1 is a transmembrane domain;
C is a costimulatory signaling domain;
CD3ζ is a cytoplasmic signaling sequence derived from CD3ζ (including wild-type or mutants/modifiers thereof);
A is a self-cleaving 2A peptide;
S is another exogenous recombinant protein;
n1 or n2 each independently is 0 or 1;
E is a signal conversion protein;
"-" is a linker or peptide bond.

3. The engineered immune cell of claim 1, wherein the signal conversion protein has a structure as shown in formula V:
L2-Z1-TM2-Z2 (V)
wherein,
L2 is absent or a signal peptide sequence;
Z1 is an extracellular domain of an immunosuppressive receptor;
TM2 is a transmembrane region;
Z2 is an intracellular domain of an immunostimulatory receptor;
"-" is a linker or peptide bond.

4. The engineered immune cell of claim 1, wherein the amino acid sequence encoding the signal conversion protein is as shown in position 523-933 of SEQ ID NO: 8, position 523-908 of SEQ ID NO: 9, position 523-936 of SEQ ID NO: 10, position 523-911 of SEQ ID NO: 11, position 937-1347 of SEQ ID NO: 16, position 937-1350 of SEQ ID NO: 17, SEQ ID NO: 19, SEQ ID NO: 20 or SEQ ID NO: 21.

5. A method for preparing the engineered immune cell of claim 1, which comprises the following steps of:
(a) providing an immune cell to be engineered; and
(b) engineering the immune cell to express the CAR molecule and exogenous signal conversion protein, thereby obtaining the engineered immune cell of claim 1.

6. A formulation comprising the engineered immune cell of claim 1, and a pharmaceutically acceptable carrier, diluent, or excipient.

7. Use of the engineered immune cell of claim 1, in the manufacture of a medicament or formulation for preventing and/or treating a cancer or tumor.

8. The use of claim 7, wherein the tumor is selected from the group consisting of: solid tumor, hematological malignancy, lymphoma, and a combination thereof.

9. The use of claim 7, wherein the tumor expresses markers selected from the group consisting of: mesothelin, Claudin18.2, MUC1, GPC3, PSCA, Her2, and a combination thereof.

10. A kit for preparing the engineered immune cell of claim 1, wherein the kit comprises a container and the following located inside the container:
(1) a first nucleic acid sequence containing a first expression cassette for expressing the CAR; and
(2) a second nucleic acid sequence containing a second expression cassette for co-expressing a signal conversion protein.

11. The engineered immune cell of claim 1, wherein the amino acid sequence of the CAR is as shown in any one of SEQ ID NOs: 8-11 and SEQ ID NOs: 16-17.
